(19) Europäisches Patentamt

European Patent Office

Office européen des brevets

(11) **EP 4 247 459 B1**

(12) **EUROPEAN PATENT SPECIFICATION**

(45) Date of publication and mention
of the grant of the patent:
**01.04.2026  Bulletin 2026/14**

(21) Application number: **21827310.0**

(22) Date of filing: **19.11.2021**

(51) International Patent Classification (IPC):
*A61M 5/315* ^(2006.01)     *A61M 5/31* ^(2006.01)

(52) Cooperative Patent Classification (CPC):
**A61M 5/31513;** A61M 2005/3131; A61M 2207/10

(86) International application number:
**PCT/US2021/060060**

(87) International publication number:
**WO 2022/109250 (27.05.2022 Gazette 2022/21)**

(54) **METHODS OF TRANSLATING STOPPERS THROUGH A TUBE**

VERFAHREN ZUR TRANSLATION VON STOPFEN DURCH EIN ROHR

PROCÉDÉS POUR TRANSLATION DE BOUCHONS À TRAVERS UN TUBE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **20.11.2020   US 202063116209 P**

(43) Date of publication of application:
**27.09.2023   Bulletin 2023/39**

(73) Proprietor: **W. L. Gore & Associates, Inc.
Newark, DE 19711 (US)**

(72) Inventors:
• **HINKLE, Tyler N.
Newark, Delaware 19711 (US)**
• **BASHAM, Robert C.
Newark, Delaware 19711 (US)**
• **MORSE, David T.
Newark, Delaware 19711 (US)**

(74) Representative: **HGF
HGF Limited
4th Floor, 1 City Square
Leeds LS1 2ES (GB)**

(56) References cited:
EP-A1- 3 058 975       EP-A1- 3 342 439
EP-A1- 3 409 311       WO-A1-2017/123835
WO-A1-2020/112612      WO-A1-2020/118275

EP 4 247 459 B1

## Description

## FIELD

The present disclosure relates generally to medical devices, such as syringes, and more particularly, to stoppers and methods of translating the stoppers through a tube.

## BACKGROUND

Medical delivery devices including syringes and pre-filled syringes function to both store and deliver drugs and or biologics (e.g., pharmaceutical and/or biopharmaceutical treatments). Pre-filled syringes generally offer cost savings to the pharmaceutical industry and may improve the safety, convenience, and efficacy of drug delivery. Biopharmaceuticals are an important class of pharmaceuticals that may benefit from the use of pre-filled syringes and related devices, such as auto-injectors. Non-limiting examples of such biopharmaceuticals include insulin, vaccines, antibodies, blood products, hormones, and/or cytokines.

Medical delivery devices of these types typically include a reservoir for receiving a liquid (e.g., a syringe barrel or a cartridge tube), a plunger rod reciprocally movable in the reservoir, and a stopper typically attached to an end of the plunger rod. Air and liquid impermeability can minimize or eliminate liquid leakage within the reservoir and can minimize or eliminate the introduction of air between an outer face of the stopper and an inner wall of the reservoir when charging or discharging the liquid inside the reservoir. A low slide force facilitates the charging and discharging of the liquid inside the reservoir. In addition to these requirements, a medical syringe, in particular, should not, adversely affect any pharmaceutical composition such as biopharmaceuticals that come in contact with the stopper.

Stoppers for use with conventional barrels and tubes are commonly made of a rubber or other elastomeric material. Although conventional stoppers may have satisfactory air and liquid impermeability, they may not have an acceptable slide force. Accordingly, silicone and/or other liquid lubricants may be applied to the outer surface of the stopper and/or the inner wall of the barrel or cartridge tube to enhance slidability of the stopper therein. However, syringes that include lubricants, such as silicone lubricants, may cause inactivation or otherwise adversely impact the efficacy of the pharmaceutical compositions.

In order to enhance low-friction slidability and stability of pharmaceutical compositions, stoppers laminated or otherwise coated with at least one layer, for example at least a fluoropolymer layer have been used. It has been observed, however, that stoppers having fluoropolymer layers may function inconsistently during their assembly and/or use. For example, the stopper may distort during insertion of the stopper into a barrel or cartridge tube, and/or during movement of the plunger rod within a barrel or cartridge tube during use. Such distortions may create leak paths for the liquid or otherwise detrimentally impact the functionality and/or appearance of the stoppers.

Therefore, there remains a need for a stopper that is air and liquid impermeable, does not distort during insertion, and which obtains an acceptable slide force. In addition, there remains a need for methods of translating the stopper through a barrel, cartridge tube, or vent or insertion tube during an assembly process and/or use of the stopper, particularly through a lubricant free or substantially lubricant free barrel, cartridge tube, and/or vent or insertion tube such that the stopper does not distort.

WO2020112612 describes a method of inserting a lubricant free stopper into a lubricant free syringe barrel or lubricant free cartridge tube, including includes (1) inserting a stopper with a sealing rib into a placement region, (2) lowering the body of the insertion tube into a syringe barrel or cartridge tube to a position located past the barrel flange or top of the cartridge tube, (3) maneuvering the stopper to a distal opening of the insertion tube using an insertion pin, (4) retracting the insertion tube while the stopper remains in a fixed position within the syringe barrel or cartridge tube, and (5) fully retracting the insertion pin and the insertion tube from the syringe barrel or cartridge tube. The method is lubricant free or substantially lubricant free. WO2020118275 describes a process for producing gaskets with an improved channel for use in matched syringe and plunger systems. A syringe comprising a syringe barrel assembled with a gasket attached to a plunger is also disclosed. EP3058975 describes a method for producing a gasket for use in a prefilled syringe. The gasket has a surface laminated with a lamination film and including a circumferential surface portion. The circumferential surface portion has formed thereon a groove and a projection, each extending circumferentially of the gasket in a portion of the lamination film. Similar apparatus is described in EP3342439 and EP3409311. WO2017123835 describes a syringe for storing and delivering a fluid that includes an elastomeric stopper and a plunger rod assembly. The plunger rod assembly includes a plunger rod and a threaded member at a plunger-engaging end. The stopper has an inner cavity and the plunger has a threaded member that contacts an engagement surface in the inner cavity.

## SUMMARY

The scope of the invention is defined by the independent claim. The dependent claims disclose further advantageous embodiments.

According to a first example, ("Example 1"), a method includes placing a distal end of a stopper on a proximal end of an insertion tube, the insertion tube and the stopper being silicone free, the stopper including a plunger rod engaging cavity and a sealing region having a length spaced from a proximal end of the stopper by a sealing location length, the sealing region having at least one rib including at least one microgroove in a polymer barrier, the at least one microgroove having an initial width and positioning an insertion pin on a proximal end of the stopper without contacting a distal region of the plunger rod engaging cavity, wherein the insertion pin has a cylindrical body that includes a distal end having a shoulder and a pin tip end that has a diameter smaller than a diameter of the plunger rod engaging cavity.

According to another example, ("Example 2"), further to Example 1, the method includes guiding the stopper through an entire length of the insertion tube and into a syringe barrel, the syringe barrel being silicone free.

According to another example, ("Example 3"), further to Example 2, during the guiding of the stopper through the insertion tube, the insertion pin engages the distal end region of the plunger rod engaging cavity.

According to another example, ("Example 4"), further to any one of the preceding Examples, during the step of placing the distal end of the stopper on the proximal end of the insertion tube, the stopper is in an uncompressed state.

According to another example, ("Example 5"), further to any one of Examples 2-4, during the step of guiding the stopper through the insertion tube, the stopper is in a compressed state.

According to another example, ("Example 6"), further to any one of the preceding Examples, applying the force on the proximal end of the insertion pin further includes transferring at least a portion of the force onto the proximal end of the stopper.

According to another example, ("Example 7"), further to Example 6, the method includes transferring at least a portion of the force onto the proximal end of the stopper includes applying one or both of (1) a first force to the proximal end of the stopper, or (2) a second force to the distal region of the plunger rod engaging cavity.

The shoulder of the insertion pin includes at least one force concentrating feature including an annular structure extending from the shoulder of the insertion pin, optionally including one of at least a flat, sharp or radiused surface configured to engage the proximal end of the stopper.

The force applied to the at least one concentrating feature such that the force applied to the stopper is at least applied to an annular surface of the proximal end of the stopper such that the force is biased towards an outer diameter of the stopper.

According to another example, ("Example 11"), further to any one of the preceding Examples, the sealing region includes a first rib and a second rib and applying the force on the proximal end of the insertion pin is such that a length between the first rib and the second rib has a reduction of increase of at least 1%.

According to another example, ("Example 12"), further to any one of the preceding Examples, the reduction of increase of the initial width of the microgroove is at least 15%.

Also disclosed is an exemplary method, not claimed ("Example 13"), of dispensing contents of a syringe barrel includes inserting a plunger rod into a proximal end of a plunger rod engaging cavity of a stopper, the stopper being silicone free, the stopper having a proximal end opposite a distal end, and a sealing region having a length spaced from the proximal end by a sealing location length, the sealing region including a polymer barrier and at least one microgroove having an initial width and positioned within the polymer barrier and contacting the proximal end of the stopper to a shoulder of the plunger rod in the syringe barrel without contacting a distal region of the plunger rod engaging cavity, the syringe barrel being silicone free and containing a therapeutic. The method further includes applying a force to the plunger rod such that a reduction of increase of the initial width of the at least one microgroove is at least 10%.

According to another example, ("Example 14"), further to Example 13, the exemplary method includes guiding the stopper through the syringe barrel through transferring at least a portion of the force applied to the plunger rod onto the proximal end of the stopper.

According to another example, ("Example 15"), further to Example 14, the shoulder of the plunger includes at least one force concentrating feature including an annular structure extending from the shoulder of the plunger rod, optionally including one of at least a flat, sharp or radiused surface configured to engage the proximal end of the stopper.

According to another example, ("Example 16"), further to Example 14, the proximal end of the stopper includes at least one force concentrating feature including an annular structure extending from the proximal end of the stopper, optionally including one of at least a flat, sharp or radiused surface configured to engage the shoulder of the plunger rod.

According to another example, ("Example 17"), further to any one of Examples 14-16, the plunger rod includes a distal end having the shoulder and a plunger rod tip, the plunger rod tip configured for being received by a distal region of the plunger rod engaging cavity of the stopper.

According to another example, ("Example 18"), further to Example 17, during the guiding of the stopper through the syringe, the plunger rod tip engages the distal region of the plunger rod engaging cavity of the stopper.

According to another example, ("Example 19"), further to Example 18, applying the force to the plunger rod includes applying one or both of (1) a first force to the proximal end of the stopper, or (2) a second force to a distal end of the plunger rod engaging cavity.

According to another example, ("Example 20"), further to any one of Examples 13-19, the sealing region of the stopper includes a first rib and a second rib and a rib length extending between the first rib and the second rib, wherein the force is applied to the plunger rod such that the rib length has a reduction of increase of at least 1%.

According to another example, ("Example 21"), further to any one of Examples 13-20, the reduction of increase of the initial width of the at least one microgroove is at least 15%.

According to another example, ("Example 22"), further to Example 13, the stopper and the plunger rod are not directly attached.

According to another example, ("Example 23"), further to any one of Examples 16-19, the at least one force concentrating feature is configured such that the force applied to the stopper is at least applied to an annular surface of the proximal end of the stopper such that the force is biased towards an outer diameter of the stopper.

According to another example, ("Example 24"), further to any one of Examples 13-23, the exemplary method further includes dispensing the therapeutic contained within the syringe barrel.

According to another example, ("Example 25"), further to any one of Examples 13-24, the stopper is in a compressed state.

## BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.

FIG. 1A is a diagrammatic cross sectional view of a syringe in accordance with some embodiments.

FIG. 1B is a diagrammatic cross sectional view of a cartridge tube in accordance with some embodiments.

FIG. 2 is a cutaway view of a stopper in accordance with some embodiments.

FIG. 3 is a cutaway view of a stopper in accordance with some embodiments.

FIG. 4 is a detailed side view of a portion of a stopper showing a rib and microgroove in accordance with some embodiments.

FIG. 5 is a detailed cross sectional view of a portion of a stopper such as that shown in FIG. 4 in accordance with some embodiments.

FIG. 6 is a detailed side view of a portion of a stopper, showing a rib and microgroove in accordance with some embodiments.

FIG. 7 is a cutaway view of a stopper in accordance with some embodiments.

FIG. 8A is a diagrammatic side view of a plunger rod in accordance with some embodiments.

FIG. 8B is an end view of the plunger rod shown in FIG. 8A in accordance with some embodiments.

FIG. 9 is a cross sectional view of a vent or insertion tube in accordance with some embodiments.

FIG. 10A is a diagrammatic isometric view of an insertion pin in accordance with some embodiments. FIG. 10B is a cross sectional side view of the insertion pin shown in FIG. 10A.

FIGS. 11A-11D are diagrammatic illustrations of a portion of a stopper and the various forces that impact the stopper during insertion of and translation through a barrel or a cartridge tube in accordance with some embodiments.

FIGS. 12A-12D are diagrammatic illustrations of portions of stoppers having therein a cavity and the various forces that may impact the stopper during insertion of and/or translation through a barrel or a cartridge tube in accordance with some embodiments.

FIG. 13A is a diagrammatic illustration of a microgroove that has a thickness that is less than the thickness of the polymer layer in accordance with some embodiments.

FIG. 13B is a diagrammatic illustration of a microgroove that has a thickness equal to the thickness of the polymer layer in accordance with some embodiments.

FIGS. 13C-13D are diagrammatic illustrations depicting the overall elongation of the stopper in a barrel or cartridge tube and associated elongation of the microgroove in accordance with some embodiments.

FIG. 14 is a cutaway view of a stopper in accordance with some embodiments.

FIG. 15 is a diagrammatic cross sectional illustration of an insertion pin in accordance with some embodiments.

FIG.16 is a diagrammatic cutaway view of the insertion pin shown in FIG. 15 engaged with a stopper in accordance with some embodiments.

FIG. 17 is a diagrammatic cross sectional illustration of an insertion pin in accordance with some embodiments.

FIG. 18 is a diagrammatic cutaway view of the insertion pin shown in FIG. 17 engaged with a stopper in accordance with some embodiments.

FIG. 19 is a diagrammatic cross sectional illustration of an insertion pin in accordance with some embodiments.

FIGS. 20A-20B are diagrammatic partial cross sectional illustrations of the insertion pin shown in FIG. 19 engaged with a stopper in accordance with some embodiments.

FIG. 21 is a diagrammatic cross sectional illustration of an insertion pin in accordance with some embodiments.

FIGS. 22A-22B are diagrammatic partial cross sectional illustrations of the insertion pin shown in FIG. 19 engaged with a stopper in accordance with some embodiments.

FIGS. 23A-23D are diagrammatic cutaway views of insertion pins including force concentrators engaged with a stopper in accordance with some.

FIGS. 24A-24D are cutaway views of stoppers including force concentrators in accordance with some embodiments.

FIGS. 25A-25D are diagrammatic illustrations of various shapes of force concentrators position on the distal end of the plunger rod in accordance with some embodiments.

## DETAILED DESCRIPTION

Definitions and Terminology

Persons skilled in the art will readily appreciate that various aspects of the present disclosure can be realized by any number of methods and apparatus configured to perform the intended functions. It should also be noted that the accompanying drawing figures referred to herein are not necessarily drawn to scale, but may be exaggerated to illustrate various aspects of the present disclosure, and in that regard, the figures should not be construed as limiting.

It is to be appreciated that the terms "barrel" and "syringe barrel" may be used interchangeably herein. In addition, the term "tube", when not used in conjunction with the term "cartridge", is meant to refer to any of a number of tubular structures into which stoppers may be inserted and/or translated through during an assembly process and/or use of syringes or cartridges, such as, but not limited to, stopper-receiving openings of transfer bars, such as flip bars or rotating bars, vent, vacuum or insertion tubes, barrels of syringes, and/or cartridge tubes of an auto-injectable device. It is to be noted that the phrase "lubricant free syringe barrel" and "lubricant free barrel" may be interchanged with the phrase "lubricant free cartridge tube" within this disclosure. Additionally, the phrases "polymer or expanded polymer layer" may be interchangeably used with the phrase "laminate layer" herein. Further, the terms "syringe" and "cartridge tube" may be used interchangeably in this disclosure. It is also to be appreciated that the term "about" as used herein denotes +/- 10% of the designated unit of measure.

Syringes

The present disclosure is directed to stoppers and methods of translating the stopper through a lubricant free or substantially lubricant free barrel, cartridge tube, and/or vent or insertion tube during an assembly process and/or use of the stopper such that the stopper does not distort or otherwise make the resultant barrel or cartridge tube containing the stopper unusable. As used herein, the term "lubricant free" is meant to denote that no liquid lubricant has been intentionally added or that the amount of liquid lubricant that may be present is below a detectable level. Lubricant free may include silicone lubricant free. Embodiments are directed to a method of moving or translating a stopper having thereon a polymer layer (e.g., a fluoropolymer or expanded fluoropolymer layer) or a laminate layer into a lubricant free or substantially lubricant free barrel or a lubricant free or substantially lubricant free cartridge tube through the use of an insertion tube and an insertion pin. Embodiments are also directed to a method of moving or translating a stopper having a body (e.g., elastomeric) and a polymer layer or a laminate layer coupled, or otherwise associated with the body, into a lubricant free or substantially lubricant free barrel or a lubricant free or substantially lubricant free cartridge tube through the use of a funneled vacuum insertion tube. Embodiments are also directed to a method of inserting a stopper into a vent or insertion tube in a transfer bar system in connection with an assembly process of a syringe or cartridge tube. Embodiments are also directed methods of moving or translating a stopper into and through a syringe barrel or cartridge tube during an assembly process and/or use of the syringe or auto-injectable device. By these embodiments the container closure integrity (CCI), acceptable slide force, and other characteristics of the syringes and cartridge tubes and their use are obtained.

The stopper may include an elastomeric body and a polymer or expanded polymer layer or laminate layer that at least partially covers the elastomeric body. As used herein, the terms "syringe" and "cartridge" are meant to refer to any device that delivers at least one therapeutic compound (e.g., drug or biologic) via injection with a needle or with a "needleless" system (e.g., a luer system) via the translation of a stopper. The syringe or auto-injector may be used to administer different therapeutic compounds such as, for example, drugs and biologics, including but not limited to, antibodies, antisense, RNA interference, gene therapy, primary and embryonic stem cells, vaccines, and combinations thereof. The disclosure hereafter equally applies to a syringe or to a cartridge and to the assembly and use of such devices. Numerous types of medical delivery devices are contemplated, such as, for example, a syringe, an auto-injector, or injectable pen, and are considered to be within the purview of the present disclosure.

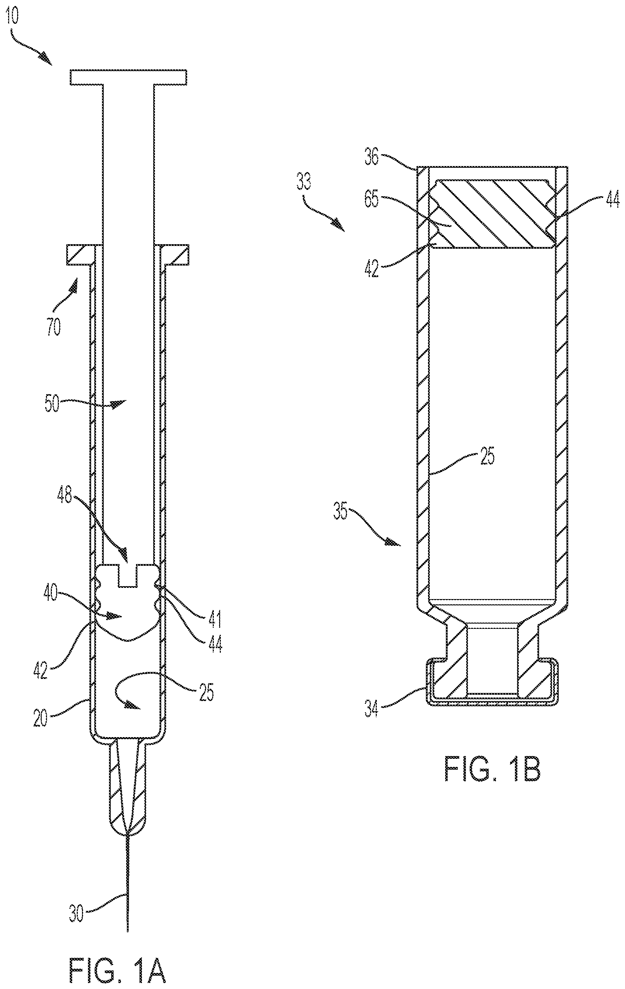

FIG. 1A illustrates a syringe 10 in accordance with some embodiments. As shown, the syringe 10 may include a barrel 20 with an inner surface 25, and a piercing element (e.g., needle) 30 or coupling (e.g., using a luer connection features) feature (not illustrated) attached thereto for injecting a therapeutic compound(s). A plunger rod 50 may include a stopper 40 affixed to a distal end of the plunger rod 50. The stopper 40 has an outer side surface 41 that contacts at least a portion of the inner surface 25 of the barrel 20 via one or more ribs 42, 44, that extend circumferentially around the stopper. Although two ribs 42, 44 are shown in FIG. 1A, any number of ribs may be present on the stopper 40. At least one of the ribs 42, 44 is a sealing rib. In some embodiments, rib 42 is the sole sealing rib. Hereafter, ribs 42, 44 will both be referred to as sealing ribs for purposes of discussion. Sealing ribs 42, 44 provide container closure integrity to the barrel 20 of a pre-filled syringe or cartridge tube of an auto-injector. The stopper 40 additionally comprises a cavity 48, also referred to herein as a plunger rod engaging cavity, that may be used for receiving the plunger rod 50, or various other translating elements, as will be described further herein. One or more flanges 70 may be used as a finger grip for pressing and translating the plunger rod 50 within the syringe barrel 20.

FIG. 1B illustrates a cartridge 33 in accordance with some embodiments. In the illustrated embodiment of cartridge 33, a modified plunger rod (not shown) and a stopper 65 are not attached. In this way, the modified plunger rod may be a floating plunger rod within the cartridge 33, such that prior to contact with the stopper 65 the modified plunger rod is floating within the cartridge 33. However, after contacting the stopper 65, the modified plunger rod is in contact with the stopper 65 during translation of the stopper 65. Although the illustrated embodiments of the stopper 65 have no cavity therein, other embodiments may include a cavity for receiving a plunger rod, for example as shown in FIG. 1A. The stopper 65 contacts at least a portion of the inner surface 25 of a cartridge tube 35 via one or more ribs, such as sealing ribs 42 and 44. The cartridge 33 contains a stopper 65, a sealed cap 34, the cartridge tube 35, and a sealing end portion 36. The sealing end portion 36 may be the open end of the cartridge 33 that may be sealed during use with the stopper 40. It is to be appreciated that the components of the syringe 10 and the cartridge 33 may be lubricant free or substantially lubricant free as described in detail below.

### Stoppers

Stopper 40 includes an elastomeric body at least partially laminated, coated, or otherwise covered by a polymer or expanded polymer layer. In some embodiments, the elastomeric body may have thereon one or more polymer or expanded polymer layers.

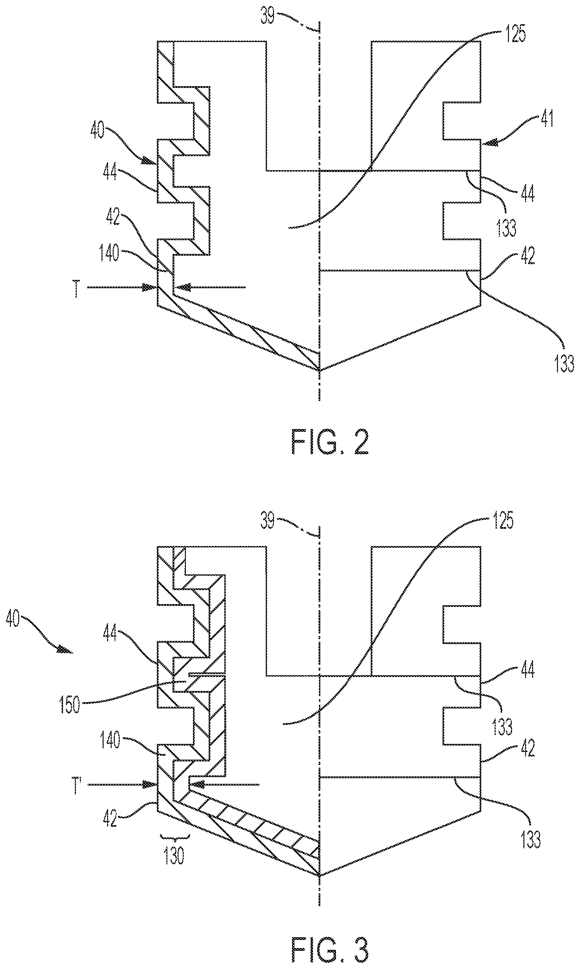

FIG. 2, for example, illustrates a stopper 40 that has an elastomeric body 125 and polymer or expanded polymer layer 140. In some embodiments, the polymer or expanded polymer layer may include a single layer of a polymer or expanded polymer that at least partially covers the elastomeric body 125. In some embodiments, the polymer or expanded polymer layer 140 encompasses or covers the elastomeric body 125. FIG. 3 illustrates other embodiments of the stopper 40 that include an elastomeric body 125 and a laminate layer 130 that may be formed of a polymer or expanded polymer layer 140 (e.g. an expanded fluoropolymer) and a porous layer 150. As discussed above, the stopper 40 may have one or more sealing ribs, such as sealing ribs 42, 44, extending therefrom. In some embodiments, ribs 42 and 44 include microgrooves 133, which is explained in detail hereafter.

In some embodiments, the polymer is a fluoropolymer, which may be expanded. Examples of fluoropolymers that may be utilized as a polymer or expanded polymer layer 140 or as the porous layer 150 include, but are not limited to, polytetrafluoroethylene (PTFE), expanded polytetrafluoroethylene (ePTFE), densified expanded polytetrafluoroethylene (ePTFE), densified polytetrafluoroethylene (PTFE), expanded modified polytetrafluoroethylene (PTFE), expanded copolymers of polytetrafluoroethylene (PTFE), ethylene-(perfluoro-ethylenepropylene) copolymer (EFEP), polyvinylidene difluoride (PVDF), fluorinated ethylene propylene (FEP), perfluoroalkoxy copolymer resin (PFA), polyvinylfluoride, perfluoropropylevinylether, and perfluoroalkoxy polymers. Patents have been granted on expandable blends of PTFE, expandable modified PTFE, and expandable copolymers of PTFE, such as, but not limited to, U.S. Patent No. 5,708,044 to Branca; U.S. Patent No. 6,541,589 to Baillie; U.S. Patent No. 7,531,611 to Sabol et al.; U.S. Patent No. 8,637,144 to Ford; and U.S. Patent No. 9,139,669 to Xu et al. Non-fluoropolymers such as polyethylene, polypropylene, or polycarbonate may also be utilized as a polymer or expanded polymer layer 140.

Non-limiting examples of elastomers that can be used to form the elastomeric body 125 include any elastomer suitable for the application, most notably rubbers constructed from butyl, bromobutyl, chlorobutyl, halobutyl, silicone, nitrile, styrene butadiene, polychloroprene, ethylene propylene diene, fluoroelastomers, thermoplastic elastomers (TPE), thermoplastic vulcanizates (TPV), and combinations and blends thereof.

The polymer or expanded polymer layer 140 may be characterized by a thickness T. In some embodiments, thickness T is a distance from about 1 $\mu$m to about 50 $\mu$m, from about 5 $\mu$m to about 40 $\mu$m, from about 5 $\mu$m to about 20 $\mu$m, or from about 20 $\mu$m to about 30 $\mu$m. The laminate layer 130 may have a thickness (T') that is less than about 30 $\mu$m. In some embodiments, the thickness of the laminate layer 130 may range from about 0.5 $\mu$m to about 20 $\mu$m, from about 0.5 $\mu$m to about 10 $\mu$m, or from about 10 $\mu$m to 30 $\mu$m. The layer forming the polymer or expanded polymer layer 140 (e.g., FIG. 2) and/or the porous layer 150 (e.g., FIG. 3) may be pretreated or post-treated with chemical etching, plasma treating, corona treatment, roughening, or the like to improve the bonding of the polymer or expanded polymer layer 140 and/or the porous layer 150 to the elastomeric body 125. The materials of the laminate layer 130 and polymer or expanded polymer layer 140 are chosen to provide a low coefficient of friction, compliance, low extractables and leachables, good barrier properties as they relate to extractables and leachables from the elastomeric body 125, as well as good air and liquid impermeability. In another embodiment, the polymer or expanded polymer layer 140 may be used with non-elastomeric materials such as, but not limited to, plastics (e.g., polypropylene, polycarbonate, and polyethylene), thermoplastics, and fluoropolymer materials such ethylene-(perfluoro-ethylene-propylene) copolymer (EFEP), polyvinylidene difluoride (PVDF), and perfluoroalkoxy polymer resin (PFA).

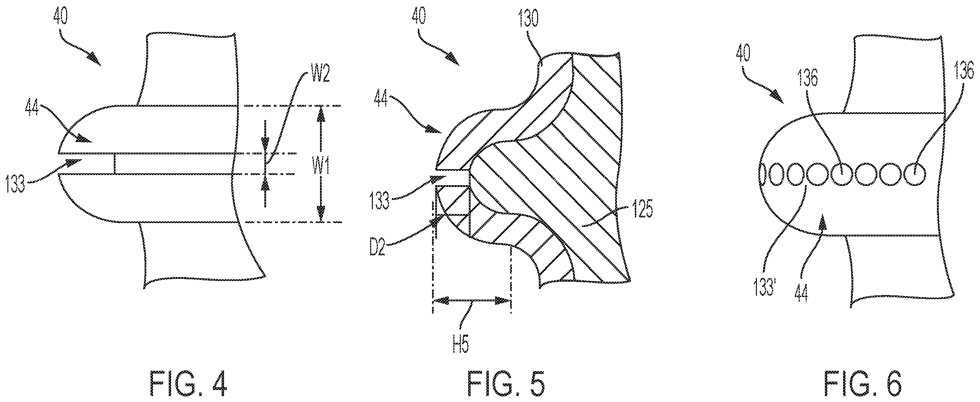

FIG. 4 is a diagrammatic illustration of a portion of a sealing rib 44 of stopper 40 in accordance with some embodiments. While the description herein is largely in reference to use of the stopper 40, the stopper 65 may be used interchangeably with the stopper 40. Although only one sealing rib 44 with a microgroove 133 is shown in FIGS. 4 and 5 for purposes of example, one or more additional sealing ribs (such as rib 42 on the stopper 40) can have the same or substantially the same microgroove 133 as sealing rib 44. As shown, rib 44 can be characterized generally as extending from a major surface area of the outer side surface of the stopper 40 by a height H5 (shown in FIG. 5) and having a width W1 (i.e., in a direction parallel to a longitudinal axis 39 of the stopper as shown in FIG. 2). In some embodiments, height H5 is a distance from about 1 $\mu$m to about 500 $\mu$m, from about 3 $\mu$m to about 400 $\mu$m, from about 200 $\mu$m to about 400 $\mu$m, or from about 250 $\mu$m to about 350 $\mu$m. Width W1 is a distance from about 5 $\mu$m to about 400 $\mu$m, from about 10 $\mu$m to about 300 $\mu$m, or from about 200 $\mu$m to about 300 $\mu$m in some embodiments. Other embodiments of stopper 40 include sealing ribs such as rib 44 having heights H5 and widths W1 that are greater or lesser distances than those described herein.

As shown in FIGS. 4 and 5, rib 44 includes a microgroove 133 (rib 42 not illustrated) that extends into the rib 44 from its outer surface. The microgroove 133 extends circumferentially around the stopper 40 within the rib 44. In FIGS. 4 and 5, microgroove 133 has a width W2 and a depth D2. Width W2 may be a distance from about 5 $\mu$m to about 300 $\mu$m, from about 5 $\mu$m to about 150 $\mu$m, from about 5 $\mu$m to about 50 $\mu$m, or from about 10 $\mu$m to about 30 $\mu$m. Depth D2 may be a distance from about 1 $\mu$m to about 50 $\mu$m, from about 5 $\mu$m to about 30 $\mu$m, or from about 5 $\mu$m to about 15 $\mu$m. In some embodiments, the depth D2 is equal to the thickness T of the polymer or expanded polymer layer 140 or T' of the laminate layer 130. The elastomer material of the elastomeric body 125 may thereby be exposed by the microgroove 133. In other embodiments, the depth D2 of the microgroove 133 is less than the thickness T of the polymer or expanded polymer layer 140 or T' of the laminate layer 130. In some embodiments, for example, the depth D2 is from about 10% to about 95%, from about 30% to about 95%, from about 50% to about 95%, or from about 75% to 95% of the thickness **T,** T' of the polymer or expanded polymer layer 140 or the laminate layer 130, respectively. Other embodiments of stopper 40 having sealing ribs 42, 44 with microgrooves 133 may have heights H5, widths W2, and depths D2 that are greater or lesser distances.

Microgroove 133 is continuous in the embodiments illustrated in FIGS. 4 and 5. In other embodiments, the microgroove 133 may be discontinuous. FIG. 6, for example, illustrates a stopper 40 having a discontinuous microgroove 133' that includes a plurality of discrete apertures 136 that may extend to depth D2 or any value lesser than depth D2. For example, apertures 136 may extend to a depth that has a value of between 5% to 100%, 10% to 80%, 15% to 70%, 25% to 60% or 35% to 50%, of depth D2. Microgrooves such as 133 and 133' can be formed by any suitable known or otherwise conventional manufacturing process, such as, but not limited to, a laser, laser ablation, or by mechanical cutting or piercing devices, such as a blade.

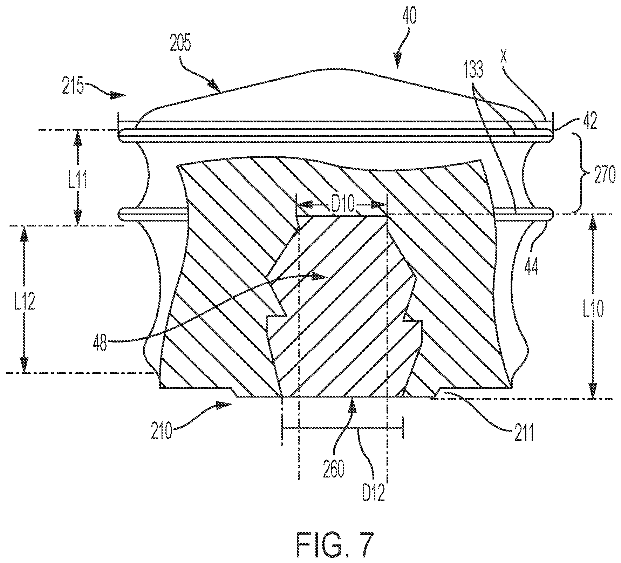

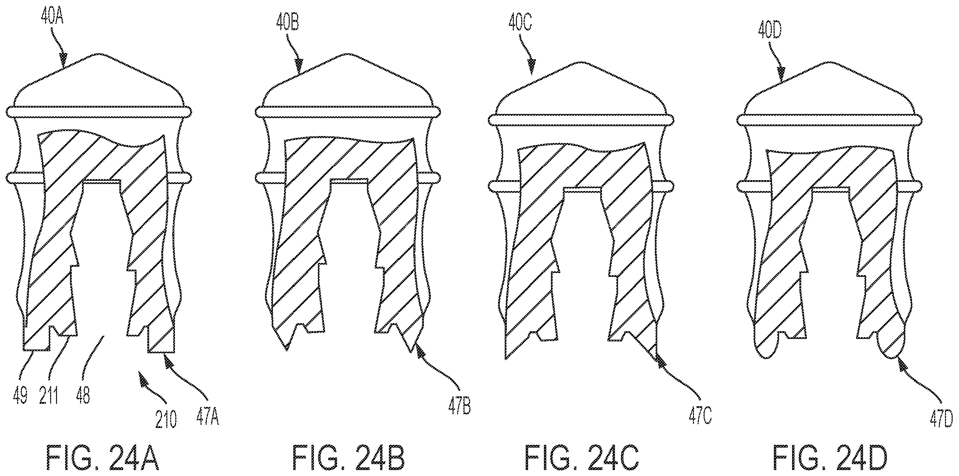

FIG. 7 illustrates various features of stoppers 40. Stoppers 40 are configured to achieve CCI with high levels of air and liquid impermeability while also maintaining acceptably low break loose and slide forces. Stopper 40 includes a body 205 having an opposed proximal end 210 and distal end 215, and ribs, such as ribs 42 and 44. At least one of the ribs 42, 44 is laminated with a polymer or expanded polymer layer 140. The cavity 48 is configured to receive a plunger rod (not illustrated) and extends a depth from the proximal end 210 of the body 205 into the body and towards the distal end 215. The proximal end 210 includes a generally annular surface 211 that extends between the cavity 48 and the outer side surface 41 (FIG. 1A). As described below, for example in connection with FIGS. 24A-24D, embodiments of the stoppers 40 may include force concentrator feature(s) on the annular surface 211 that cooperate with other processing or actuation components, such as, but not limited to the insertion rod, the transfer rod, and/or the plunger rod assembly.

As the skilled artisan will appreciate, ribs 42, 44 can be structured in any number of configurations, and FIG. 7 is provided for purposes of illustration only, and is not intended to limit the present disclosure. For example, in some embodiments, all of the ribs 42, 44 may have the same pre-defined outer diameter (x). In other embodiments, each rib 42, 44 may have its own predefined outer diameter (x). For example, a distal or leading rib such as 42 may have a predefined outer diameter (1x) and rib 44 may have a predefined outer diameter (2x) that is between about 75% and about 99.9%, between about 80% and about 95%, or between about 85% and about 90% of the predefined outer diameter (1x).

As shown in FIG. 7, ribs 42, 44 define a sealing feature region 270, also referred to as a sealing region 270, having a length L11. The sealing feature region 270 is spaced from the proximal end 210 of the stopper 40 by a sealing location length L12. The distance between a proximal most sealing rib (44 in the illustrated embodiment) and a distal most sealing rib (42 in the illustrated embodiment) may be characterized as total rib length L11. In these embodiments, as there are two sealing ribs 42, 44, the total rib length L11 is interchangeable with the sealing region length L11. However, in various other embodiments wherein there may be additional sealing ribs may be incorporated, the total rib length may not be equal to the length L11 of the sealing region 270.

Embodiments of the cavity 48 can be described with reference to FIG. 7. As shown, the cavity 48 is a recess having an opening 260 in the proximal end 210 and the annular surface 211 of the proximal end 210 of the stopper 40.

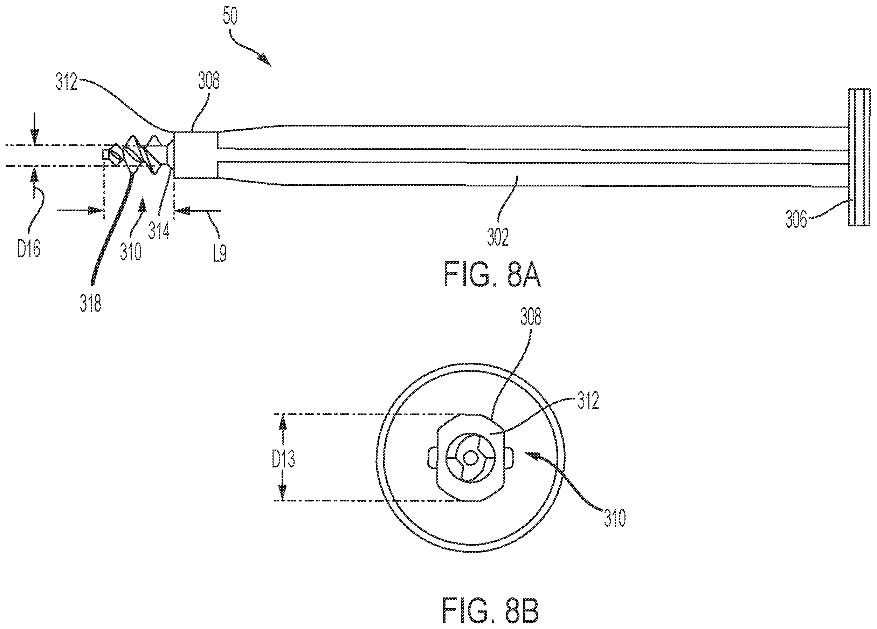

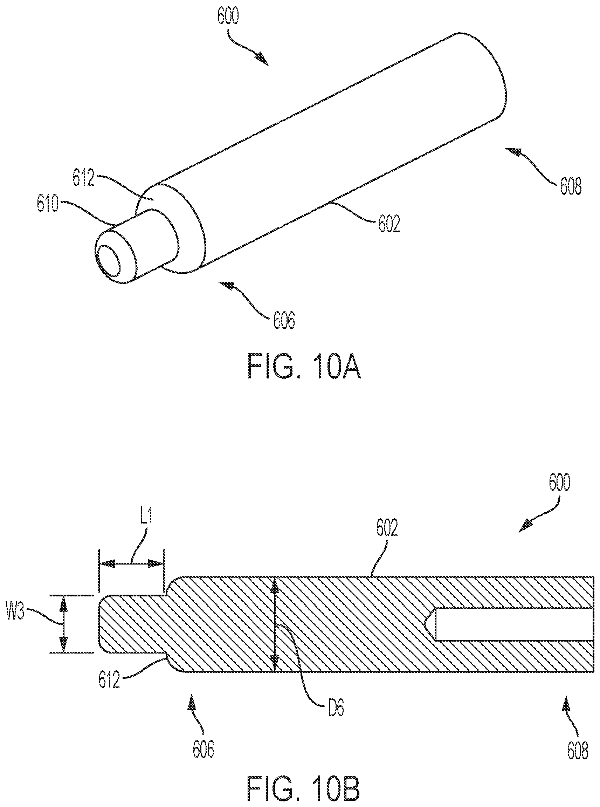

The cavity 48 is sized and configured to receive the tip of a transfer bar insertion pin, which may be used with a transfer bar system for the assembly process, the pin tip end 610 of an insertion pin 600 (described below in connection with FIG. 10A) and/or the tip 310 of the plunger rod 50 (described below in connection with FIGS. 8A and 8B) during the assembly process and/or use of the syringe 10 or cartridge tube 35. The cavity has a length L10, a diameter D10 at a distal end portion of the cavity 48 and a diameter D12 at a proximal end portion of the cavity 48. In some embodiments, for example, the length L10 of the cavity 48 may be from about 2.0 mm to about 7.3 mm, from about 3.7 mm to about 6.0 mm, or from about 4.2 mm to about 5.0 mm. The diameter D10 may, for example, be from about 1.0 mm to about 3.0 mm, from about 1.0 mm to about 1.5 mm, from about 1.3 mm to about 2.1 mm, or from about 1.6 mm to about 1.9 mm. The diameter D12 of the cavity 48 may, for example, be from about 2.3 mm to about 11.0 mm, or from about 2.2 mm to about 2.4 mm.

## Plunger Rod

FIGS. 8A and 8B illustrate non-limiting examples of a plunger rod 50 in accordance with some embodiments. As shown, the plunger rod 50 includes a body 302 including a proximal end portion 306 and a distal end portion 308. A tip 310 of the plunger rod 50 is coupled to the body 302 at the distal end portion 308 and is configured to engage the cavity 48 of the stopper 40 (shown for example in FIG. 7). The body 302 and the tip 310 have diameters smaller than the inner diameter of the barrel 20 to enable the plunger rod 50 to be received by and advanced or translated though the barrel 20. The proximal end portion 306 can be engaged by a user of the syringe 10 to actuate the plunger rod 50.

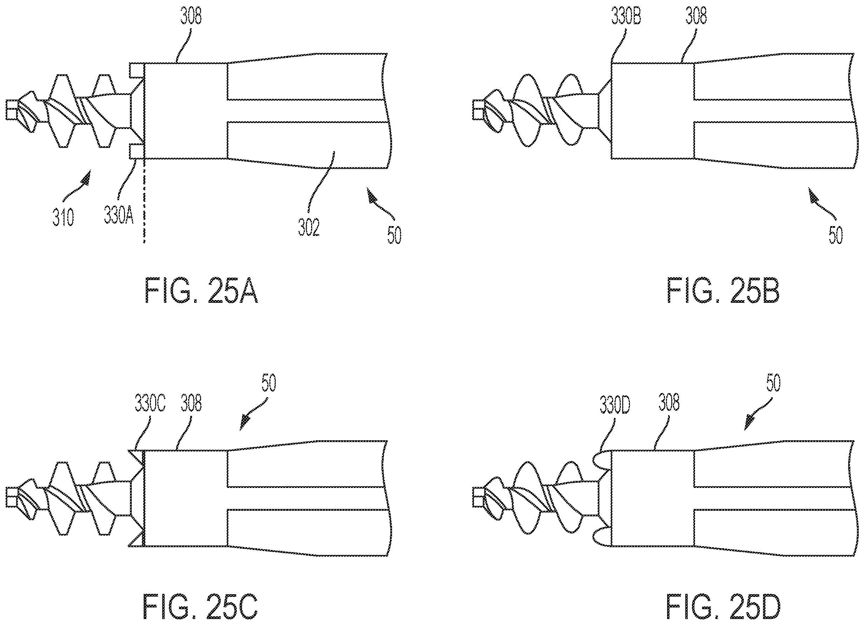

The distal end portion 308 of the plunger rod 50 defines a shoulder 312 which has a diameter D13. The tip 310 includes an engagement portion 314 coupled to the shoulder 312 (FIG. 8B). The tip 310 may have a length L9. Some embodiments such as those illustrated in FIGS. 8A and 8B include force concentrator(s), such as is illustrated, for example, in FIGS. 25A-25D, described in detail below. Engagement features such as but not limited to helical threads 318 extend from the engagement portion 314 to engage the tip 310 to a proximal end of a stopper in an assembled syringe 10 or cartridge tube 35. The engagement portion 314 taper from a first diameter to a smaller diameter D16 with increasing distance toward the distal end portion 308 in the illustrated embodiments. In other embodiments, the engagement portion 314 has a relatively constant diameter. The above described embodiment of a plunger rod 50 are non-limiting and variations thereof may be incorporated in the present disclosure.

## Syringe Assembly Process and Use of Stopper

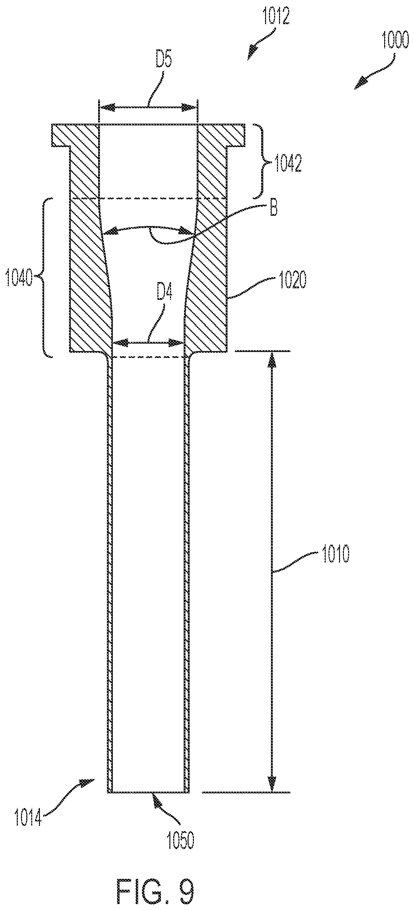

Various methods may be used for inserting a lubricant free or substantially lubricant free stopper into a lubricant free cartridge tube in accordance with embodiments. For example, in some embodiments, a vent or insertion tube 1000 as shown in FIG. 9 may be used in combination with an insertion pin as shown in FIGS. 10A and 10B. In some embodiments, for example, insertion tube 1000 enables a lubricant free stopper 40 to be placed inside a lubricant free syringe barrel or lubricant free cartridge tube without over-pressurizing the liquid contained therein. As shown, the insertion tube 1000 has a proximal end 1012 and a distal end 1014. The insertion tube 1000 also includes a body 1010 and a machine adaptor 1020. The body 1010 is the portion of the insertion tube 1000 that fits within a syringe barrel such as 20 and allows a stopper such as 40 to be placed into a syringe barrel or cartridge tube (not illustrated). The transition zone 1040 is a region where the stopper 40 is compressed to a diameter D4 that is sufficient to pass through the distal opening 1050 of the insertion tube 1000. As such, the stopper 40 is compressed from an uncompressed state to a compressed state as it is translated through the transition zone 1040.

Thus, the diameter of the stopper 40 (not shown in FIG. 9) is reduced from D5 (i.e., the diameter of the placement region 1042 or about the diameter of the stopper in the non-compressed state) to D4 (i.e., the diameter at the distal end of the transition zone 1040). The transition zone 1040 tapers from the placement region 1042 to the body 1010 at a taper angle B. In some embodiments, for example, the placement region 1042 has a diameter D5 from about 3 mm to about 20 mm, about 5 mm to about 15 mm, or from about 7 mm to about 10 mm. In some embodiments, the taper angle B may range from about 1 degree to about 20 degrees, from about 5 degrees to about 20 degrees, from about 1 degree to about 15 degrees, from about 1 degree to 10 degrees, from about 4 degrees to about 8 degrees, or from about 5 degrees to about 10 degrees.

FIG. 10A and FIG. 10B illustrate an example of an insertion pin 600 in accordance with some embodiments that may be used in combination with an insertion tube, such as the insertion tube 1000, to insert a stopper such as the stopper 40 into a barrel 20 or the cartridge tube 35. The insertion pin 600 includes a cylindrical body 602 comprising a distal end 606 and a proximal end 608. The pin tip end 610 of insertion pin 600 is connected to and extends from a shoulder 612 on the cylindrical body 602, and interfaces with the cavity 48 of the stopper 40 during assembly of the syringe 10. The proximal end 608 may be sized to mate with a machine adapter (not shown) used to push and translate the insertion pin 600 through an insertion tube, such as the insertion tube 1000 shown in FIG. 9.

The cylindrical body 602 has a diameter that is slightly smaller than the inner diameter of the body of an insertion tube (e.g., diameter D4 of the insertion tube 1000 shown in FIG. 9). The shoulder 612 is radiused and is designed to push against the surface on the proximal end of a stopper, for example the stopper 40, during the insertion of the stopper 40 into the syringe barrel 20. In other embodiments, the shoulder 612 may include or be formed of shapes other than a radius, such as but not limited to, for example, a flat surface, a linear taper, curvilinear, rounded, or have multiple tapers, or may incorporate force concentrators as will be described further herein. As described below, embodiments of the insertion pin 600 may include force concentrators on the shoulder 612 that cooperate with the proximal end of stoppers during the assembly of syringes and cartridge tubes. In these embodiments, the force concentrators of the shoulder 612 may include or be formed of shapes including, but not limited to, a flat surface, a radiused surface, a linear taper, curvilinear, rounded or with multiple tapers.

The pin tip end 610 has a length L1. In some embodiments, length L1 can be zero, greater than zero but less than the depth L10 of the cavity 48 of the stopper 40, approximately the depth L10 of the cavity 48, or greater than the depth L10 of the cavity (see FIG. 7). In some embodiments, for example, the pin tip end 610 has a length L1 generally from about 1 mm to 12 mm, 1 mm to 10 mm, 2 mm to 9 mm, 3 mm to about 8 mm, from about 4 mm to about 7 mm, from about 4.5 mm to 5.5 mm, or from about 5 mm to about 6 mm.

## Stopper Deformation During Assembly Process and Use of Stopper

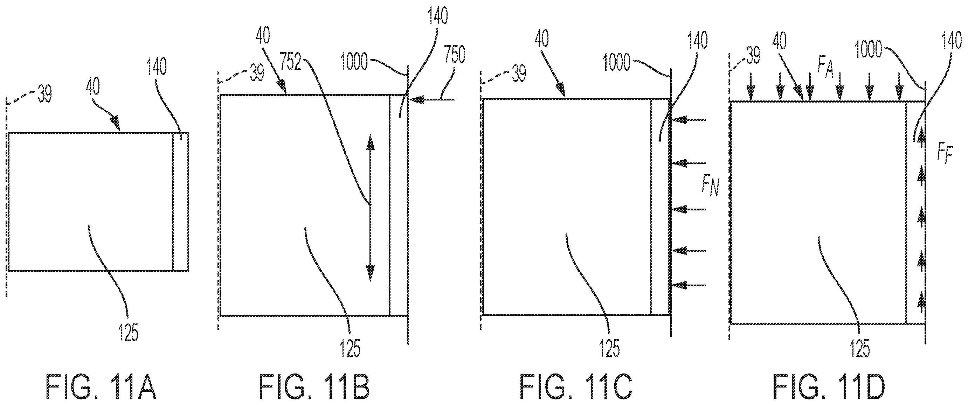

The effects of the compression of the stopper 40, and in particular certain physical effects on the elastomeric body 125 and/or the polymer or expanded polymer layer 140 (or laminate layer 130 (not depicted)), can be described with reference to FIGS. 11A-11D, 12A-12D, and 13A-13D. The following descriptions of FIGS. 11A-11D, 12A-12D, and 13A-13D, and descriptions throughout this specification, use terms such as "tube," and an associated "translating element." As noted herein, these terms are used to describe a number of different components of the syringe 10 and the manufacturing equipment (nor illustrated) used to assemble the syringes 10. For example, the plunger rod 50 is a "translating element" that may be used to translate a stopper 40 through a "tube" in the form of the syringe barrel 20 or the cartridge tube 35 during the assembly process and/or use of the syringe 10. As another example, a vacuum funnel or vacuum assist funnel may be used in conjunction with a pin that may be a "translating element" used to translate a stopper 40 through a "tube" during the assembly process and/or use of the syringe 10. As another example, the insertion pin 600 is a "translating element" that may be used to translate a stopper 40 through a "tube" in the form of an insertion tube 1000 during the assembly process of the syringe 10. As yet another example, a transfer bar insertion pin is a "translating element" that may be used to translate a stopper 40 through a "tube" in the form of stopper-receiving opening during the assembly of the syringe 10. Those of skill in the art will understand that other tubes and translating elements may be used in connection with the assembly and use of syringes 10, such as, for example, with vacuum insertion engagement tubes, and the descriptions herein apply to such other tubes and translating elements as well.

Turning to FIG. 11A, a portion of stopper 40 including an elastomeric body 125 and a polymer or expanded polymer layer 140 before the stopper 40 is compressed is depicted. The stopper 40 may be compressed in various ways, including but not limited to, in a vent tube, in a barrel, in a vacuum funnel, in a transfer bar, or any other suitable mechanism for compression. FIG. 11B is a diagrammatic illustration showing the radial compression (represented by arrow 750) of the portion of the stopper 40 during and following an assembly process, for example after insertion into an insertion tube 1000. Although described with reference to the insertion tube 1000 in connection with FIGS. 11A-11D, 12A-12D, and 13A-13D, similar effects are produced during other manufacturing steps and use of the syringe 10 as described herein. As indicated by the arrow 752, the compression of the stopper 40 may cause elongation of portions of the stopper 40 in a direction generally parallel to the longitudinal axis 39 of the stopper 40 and perpendicular to the compressive forces applied by the insertion tube 1000. The radial compression causes normal forces FN illustrated diagrammatically in FIG. 11C.

FIG. 11D illustrates certain effects on the stopper 40 when the stopper 40 is driven and translated through a tube such as an insertion tube 1000. An applied force FA is required to overcome a frictional force FF between the portions of the stopper 40 engaging the inner surface of the insertion tube 1000 (e.g., sealing ribs 42, 44), to enable the stopper 40 to slide in the tube (e.g., insertion tube 1000). The applied force (FA) may be defined by the following equation:

$$F_A = \mu F_N,$$

where $\mu$ is the friction coefficient between the polymer or expanded polymer layer 140 and the inner surface of the insertion tube 1000.

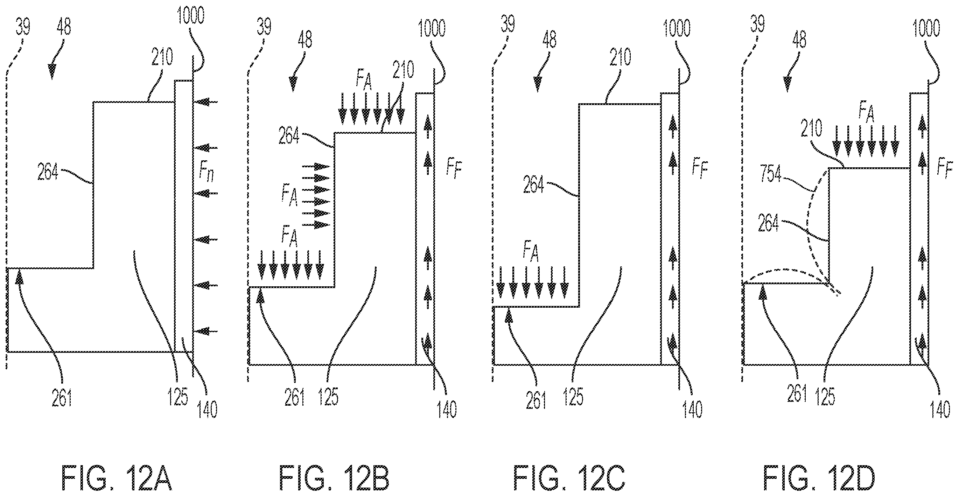

FIG. 12A is a diagrammatic illustration similar to that of FIG. 11C, and illustrates the normal forces FN caused on a radially compressed portion of the stopper 40 which includes a cavity 48, thereby creating a non-uniform geometry with respect to the geometry of the structure shown in FIG. 11C. FIG. 12B diagrammatically illustrates an applied force FA used to overcome a frictional force FF between the portions of the stopper 40 engaging the inner surface of the insertion tube 1000 to enable the stopper 40 with the cavity 48 to slide in a tube, such as an insertion tube 1000 in accordance with some embodiments. For purposes of illustration, FIG. 12B shows a uniform applied force FA to a surface at the distal end portion 261 and side wall 264 of the cavity 48, and to the annular surface 211 at the proximal end 210 of the stopper 40. The force FA can be applied for example, by a pin tip end of an insertion pin when the stopper 40 is being driven and translated through the insertion tube 1000.

FIG. 12C diagrammatically illustrates an embodiment where an applied force FA is applied only to the distal end portion 261 of the cavity 48 (e.g., in the illustrated example, no forces are applied to the side wall 264 or surface of the proximal end 210 of the stopper 40). As shown, the applied force $F_A$ causes the elastomeric body 125 to elongate.

FIG. 12D diagrammatically illustrates the applied force FA applied to the surface at the proximal end 210 of the stopper 40 (e.g., in the illustrated example, no forces are applied to surfaces of the cavity 48 such as the distal end portion 261 and/or side wall 264). As described in greater detail below, stretch, elongation, or other distortion of the polymer or expanded polymer layer 140 and/or the microgrooves 133 may be controlled by such applied forces FA. The frictional force FF with an applied force FA shown in FIG. 12D may be less than a frictional force FF with a uniform applied force FA shown in FIG. 12B. As indicated by the broken lines 754 in FIG. 12D, by the applied force FA in the illustrated example, the elastomeric body 125 tends to expand toward or into the cavity 48 under this force arrangement, reducing the normal force FN (illustrated in FIG. 12A).

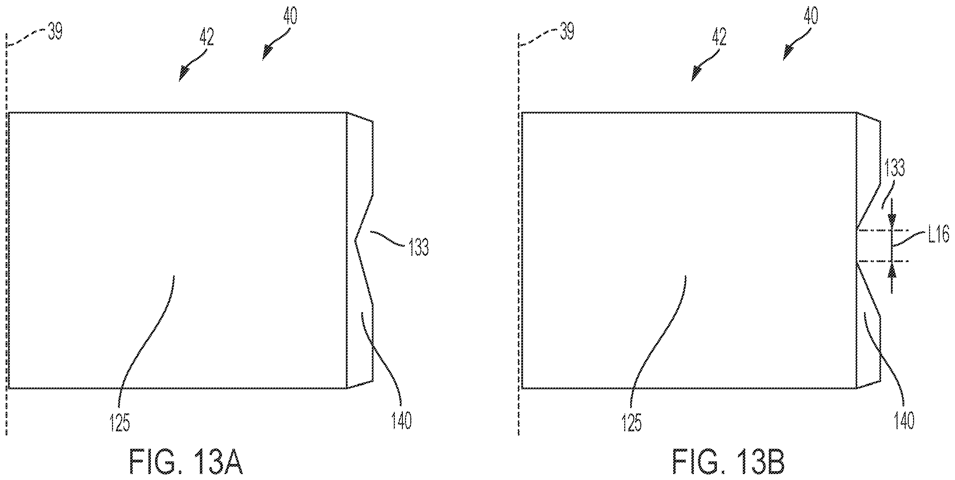

Turning to FIGS. 13A-13B, a portion of a rib of the stopper 40 having a microgroove 133 therein is illustrated, with the stopper 40 in its non-compressed state. In the non-compressed state, the stopper 40 has not been introduced into any of the "tubes" used for compression, as were previously described. The microgrooves 133 define thinned or discontinuous grooves and/or regions in the polymer or expanded polymer layer 140 at the locations where the rib 42 engages and is compressed by a tubular structure (e.g., insertion tube 1000). FIG. 13A is a diagrammatic illustration of a rib 42 that includes the microgroove 133 in the polymer or expanded polymer layer 140 before the rib 42 (or stopper containing the rib 42) is compressed. The maximum depth of the microgroove 133 illustrated in FIG. 13A (which, as shown, has sloping side walls and a maximum depth at about its center) is less than the thickness of the polymer or expanded polymer layer 140, and no portion(s) of the elastomeric body 125 is exposed on the outer surface of the polymer or expanded polymer layer 140 (with the possible exception of elastomeric material of the elastomeric body 125 being present at the maximum depth portion of the microgroove 133). FIG. 13B is a diagrammatic illustration of a rib 42 that includes a microgroove 133, which, as depicted, has sloping side walls and a maximum depth at least as great as the thickness of the polymer or expanded polymer layer 140. The stopper 40 is shown in its non-compressed state in FIG 13B. A length L16 of the elastomeric material of the elastomeric body 125 of the stopper 40 is exposed within the microgroove 133 in the example shown in FIG. 13B.

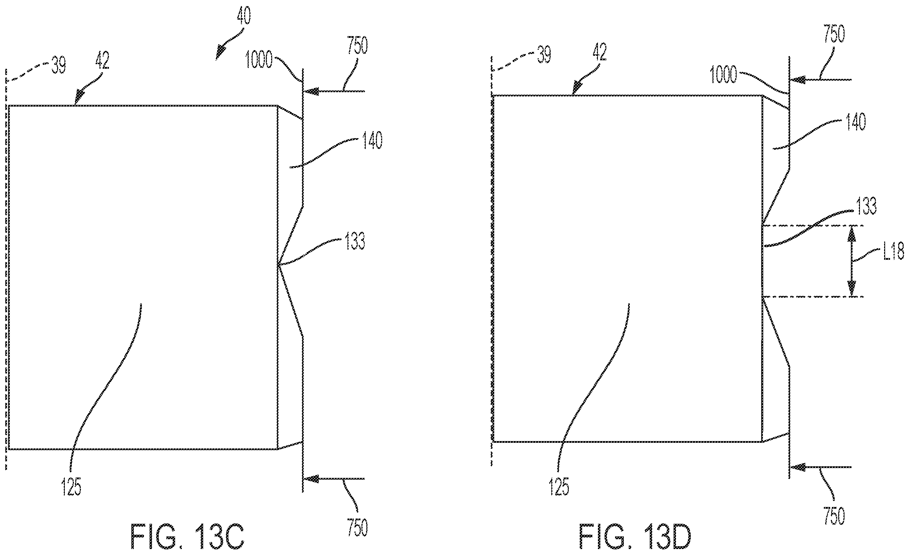

The stopper 40 is shown under radial compression (represented by arrow 750), for example from insertion in a vent tube, barrel, cartridge, transfer bar, or any other suitable compression mechanism, which may cause elongation of portions of the stopper 40 in a direction generally parallel to the longitudinal axis 39 of the stopper 40 and perpendicular to the compressive forces applied by the tube (e.g., insertion tube, barrel, or cartridge tube). As illustrated diagrammatically in FIGS. 13C and 13D, the elongation of the stopper 40, and thus the rib 42, may result in elongation and thinning of the polymer or expanded polymer layer 140. In these embodiments, if the rib 42 comprises the microgroove 133, an opening of the microgroove 133 may increase in width (for example, width W2 as shown in Fig. 4), and as such, may decrease in depth. FIG. 13C, for example, shows the thinning of the polymer or expanded polymer layer 140 with respect to the thickness shown in FIG. 13A by an amount that causes the maximum depth of the microgroove 133 to be about equal to the thickness of the polymer or expanded polymer layer 140. FIG. 13D shows the thinning of the polymer or expanded polymer layer 140 with respect to the thickness shown in FIG. 13B. By the elongation and thinning illustrated in FIG. 13D, the length L16 of the portion of the microgroove 133 having a depth at least as great as the thickness of the polymer or expanded polymer layer 140 (and where the elastomeric material of the elastomeric body 125 is exposed) may increase to a length L18 that is greater than the length L16.

FIGS. 13C and 13D illustrate the ribs 42 of the stoppers 40 in compressed states having microgrooves 133 that are free or substantially free of the elastomeric body 125 material for purposes of example (e.g., the elastomeric material has not expanded, bloomed or otherwise taken presence in the area defined by the microgroove 133).

It has been observed that during assembly and/or use of stoppers such as stoppers 40 in syringes 10, the frictional and/or other forces acting on the polymer or expanded polymer layer 140 and/or elastomeric body 125 material can cause additional deformation of the polymer or expanded polymer layer 140 and/or elastomeric body 125 material (e.g., in addition to the deformation illustrated and described in connection with FIGS. 13C and 13D). These frictional and/or other forces may also cause tensile or other failures in the polymer or expanded polymer layer 140. Observed deformations, for example, include buckling and wrinkling of the outer side surface 41 of the stoppers 40, including at ribs such as 42 and 44. Forces such as these can, for example, be produced by the compression of the stoppers 40 from their non-compressed states to their compressed states, and/or by the translation or sliding motion of the stoppers in the tubes (i.e., in a direction parallel to the longitudinal axis of the tube). These deformations and/or failures of the polymer or expanded polymer layer 140 and/or the elastomeric body 125 may result in deformation, for example an increase in width, of the ribs 42, 44 and/or microgroove 133 during the manufacturing processes. Similarly, these deformations and/or failures of the polymer or expanded polymer layer 140 and/or elastomeric body 125 may result during use of the syringe or auto-injector.

It has been observed that by any or all of these deformations and/or failures, the elastomeric body 125 may bloom, expand into or otherwise take a presence in the microgroove 133. By these deformations and/or failures, the elastomeric body 125 may expand into contact with the inner surface 25 of the tube. Similarly, portions of the polymer or expanded polymer layer 140 may peel or tear away from the elastomeric body 125, and tear or otherwise break resulting in undesired openings in the polymer or expanded polymer layer 140. The elastomeric body 125 material may extend through such undesired openings and present the elastomeric material at the inner surface of the tube. Deformations and/or failures of these types may be particularly problematic during the translation of the stoppers 40 in the tubes. These effects may be enhanced when the portions of the elastomeric body 125 that contacts the inner surface 25 of the tube is lubricant free or substantially lubricant-free because of factors such as increased frictional forces.

Engagement of portions of the elastomeric body 125 of stoppers 40 with the inner surface of the tubes during the assembly process and/or use of syringes 10 can detrimentally impact the functionality of the associated syringes 10. For example, any gaps between the exterior surface of the stoppers 40 and the inner surface 25 of the barrel 20 of the syringes 10 may detrimentally impact the container closure integrity (CCI). The therapeutic compounds in the syringes 10 may be exposed to undesirable substances such as air or other gasses, or particulates, which may detrimentally impact the therapeutic compounds. Increases in the frictional forces between the stoppers 40 and the inner surfaces 25 of the syringe barrels 20 may detrimentally impact the operation of the syringes 10 by, for example, increasing the break-loose and sliding forces of the syringe 10 (or cartridge tube 35). These impacts on operation may lead to non-injection or particulation of the elastomer, and thus contamination, and as such may cause shut down of the assembly machines.

Syringe Enhancement Examples

Structures and methods disclosed herein may enhance the functionality of lubricant free, or substantially lubricant free, syringes. For example, the CCI of the syringes may be increased. Exposure of the therapeutic compounds in the syringe barrels to undesired substances such as gasses, leachables, reactants, or other materials, e.g., such as the underlying body of the stopper, that would give rise to unwanted interactions with the syringe barrel contents, can be reduced or minimized. The break loose and slide forces of the syringes during use can be reduced or minimized.

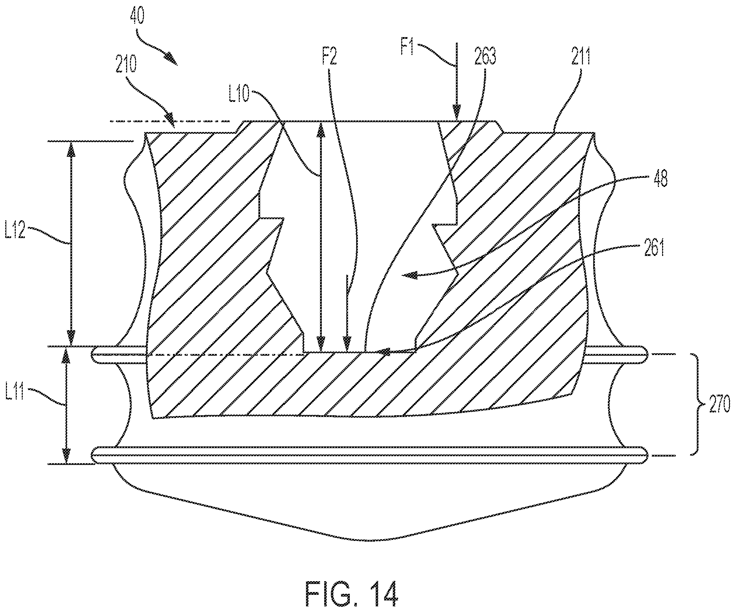

FIG. 14 illustrates certain translational forces that may be applied to the stopper 40 during the assembly and use of a syringe 10. As shown, the translational forces may include components such as F1 applied to the proximal end 210 of the stopper 40, such as, for example, to the annular surface 211, and components such as F2 applied to the distal end portion 261 of the cavity 48, such as, for example, the distal end surface 263. The structures and methods disclosed herein contemplate the use and/or application of translation forces such as components F1 and/or F2 to the stopper 40 during syringe assembly and/or use sufficient to translate the stopper through the tube (e.g., transfer-bar, insertion tube barrel 20, and/or cartridge tube 35) in a compressed state with a reduction in the deformation of the stopper 40. Specifically, the stopper 40 may be translated with a reduction of increase of a length between any two sealing ribs of the stopper 40. For example, applying translation forces such as components F1 and/or F2 to the stopper 40 during syringe assembly with the structures and methods herein may cause a reduction of increase of a sum of the sealing region length L11 and the sealing location length L12, compared to the increase in the sum of the sealing region length L11 and the sealing location length L12 that occurs during the use of conventional translation methods. For example, the sum of the sealing region length L11 and the sealing location length L12 may exhibit a reduction of increase of at least 1% of the sum of the length L11 of the sealing region 270 and the sealing location length L12 exhibited during the use of conventional methods. In various embodiments, the reduction of increase may be at least 3% of the increase of the sum that occurs during the use of conventional translation methods, and in further embodiments, the reduction of increase may be at least 5% of the sum that occurs during the use of conventional translation methods. While described throughout as a reduction of increase in the sum of the length L11 of the sealing region 270 and the sealing location length L12, the reduction of increase in length may be in reference to any length between various ribs of the stopper or the proximal end 210 and the distal end 215 of the stoppers 40.

Additionally, the use of the structures and methods described herein may also reduce the increase in size of the microgroove 133, or close the microgroove 133 entirely, during compression. Specifically, the use of the structures and methods herein may reduce the increase in the width W2 (FIG. 4) of the microgrooves 133 while the stopper 40 undergoes syringe assembly. For example, the reduction of increase in the width W2 of the microgrooves 133 may be at least 10% of the increase of the width that results from the use of conventional translation methods. In various embodiments, the reduction of increase may be at least 15% of the increase of the width that results from the use of conventional translation methods. Each of these results arise from the translational forces on the stopper 40 being more biased towards the outer diameter of the stopper 40 through the use of force concentrators, as will be described further herein. Alternatively, or in addition to, in some embodiments, the length L11 of the sealing region 270 has a reduction in increase in comparison to when these methods and structures are not used. By these structures, for example the force concentrators, and methods described above, the structures within the sealing region 270 collapse, either partially or fully, to prevent deformations to the stopper 40 and/or its polymer or expanded polymer layer 140 that might otherwise cause portions of the elastomeric material to be exposed, more exposed, bloom or otherwise contact the inner surfaces of the tubes during the assembly and/or use of the syringes 10. The collapse of the structures within the sealing region 270 may also either partially or fully prevent an increase in size, for example the width W2 (FIG. 4), of the microgrooves 133. For example, by the approaches described herein, the microgrooves 133 may close to prevent the elastomeric body 125 material from taking a presence in the microgrooves. While rib 44 is used as a sealing rib for determining the sealing location length L12, in various other embodiments the sealing rib may be a different rib of the stopper 40. For example, the stopper 40 may comprise additional ribs positioned along the stopper 40 that may be alternatively used as a sealing rib for determining sealing location length L12. Similarly, various other ribs may be used in place of rib 42 for determining length L11, sealing region 270, and sealing location length L12.

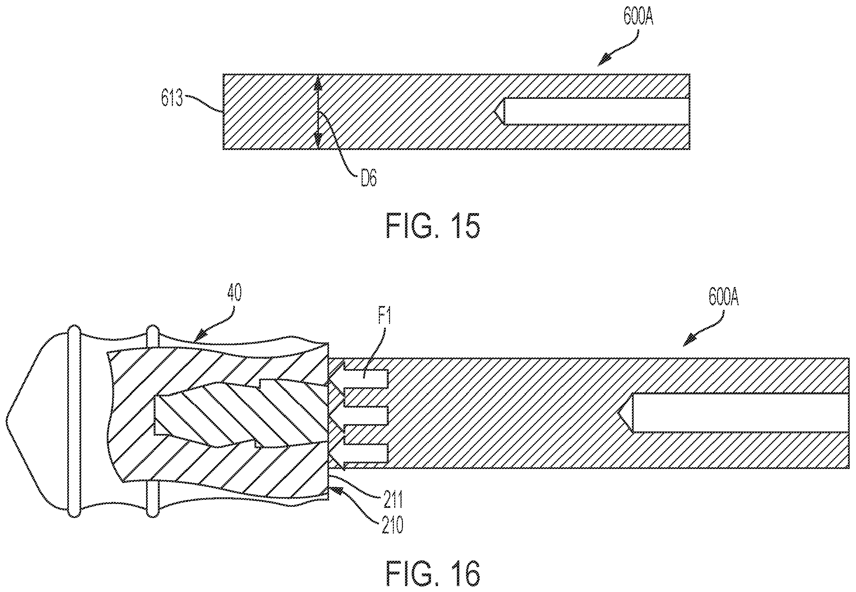

FIG. 15 illustrates a side cutaway view of an insertion pin 600A that may be used in conjunction with a tube for assembly of the stopper 40, for example as shown in FIG. 16. The insertion pin 600A comprises a shoulder 613 defined by a diameter D6. With reference to both FIGs. 15 and 16, the shoulder 613 engages with the annular surface 211 of the stopper 40 during translation of the stopper 40. The translation forces F1 are thereby applied to the proximal end 210 of the stopper 40 and against the annular surface 211 of the stopper 40. In this way, the force is concentrated to the outer diameter of the cavity 48 of the stopper 40. No force components such as those identified as F2 in FIG. 14 are applied to the stopper 40 by the insertion pin 600A shown in FIGS. 14 and 15. However, as the translation forces F1 are biased to the outer diameter of the cavity 48 through the engagement with the annular surface 211, the deformation of the stopper 40 may be reduced. Specifically, a width of the microgrooves 133 of the stopper 40 may have a reduced increase after translation, and/or the sum of the length L11 of the sealing region 270 and the sealing location length L12, and/or the length L11 of the sealing region 270 may have a reduced increase during translation as well.

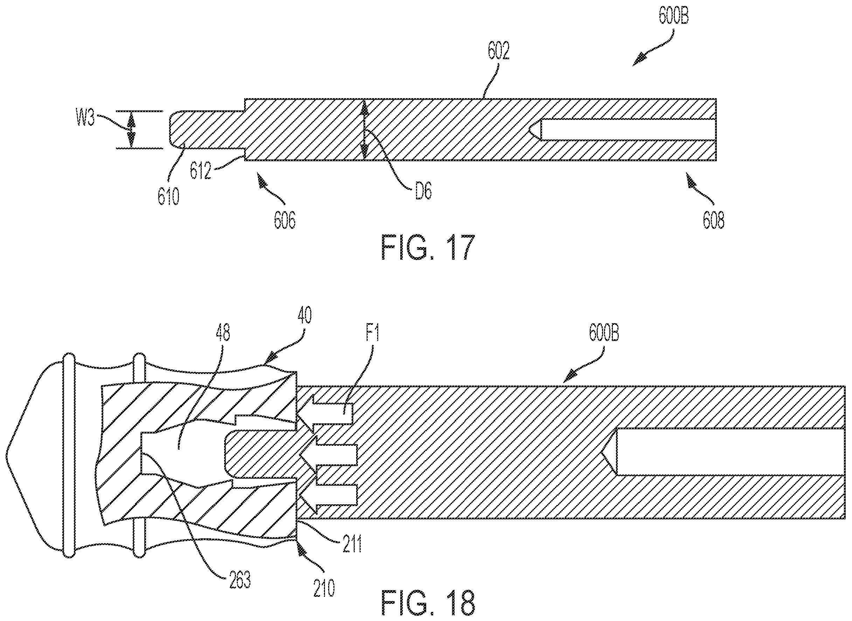

With reference now to FIGs. 17 and 18, an insertion pin 600B may be used for the translation of the stopper 40. The insertion pin 600B comprises a shoulder 612 defined by the diameter D6, similar to the shoulder 613 of insertion pin 600A (FIG. 15), and also comprises a pin tip end 610 extending from the shoulder 612. During use with the stopper 40, the pin tip end 610 is thereby located within the cavity 48 without engaging the distal end portion 261 or distal end surface 263, when the surface of the shoulder 612 of the insertion pin 600B engages the annular surface 211 of the stopper 40. Translation forces F1 are thereby applied to the proximal end 210 of the stopper 40. No force components such as those identified as F2 in FIG. 14 are applied to the stopper 40 by the insertion pin 600B shown in FIGS. 17 and 18. During the insertion of insertion pin 600B into the cavity 48 of the stopper 40, and/or the translation of the insertion pin during the assembly of the syringe 10, the pin tip end 610 enhances the alignment of the stopper and insertion pin. Although described in connection with an insertion pin 600B used to translate the stopper 40 through an insertion tube such as 1000, embodiments include a transfer bar insertion pin having a distal end portion and a flat surface such as those of insertion pin 600B, and the use of such a transfer bar insertion pin during the assembly of a syringe 10.

By the use of the structures and methods described in connection with FIGS. 17 and 18, the stoppers 40 can be translated through tubes during the assembly of the syringe 10 with a reduction of increase of the width W2 (FIG. 4) of the microgrooves 133. For example, the reduction of increase in the width W2 of the microgrooves 133 may be at least 10% of the increase of the width that occurs during the use of conventional translation methods. In various other embodiments, the reduction of increase may be at least 15% of the increase of the width that occurs during the use of conventional translation methods. Additionally, by the use of the structures and methods described herein, the stoppers 40 can be translated through tubes during the assembly with a reduction of increase in the sum of the length L11 of the sealing region 270 and the sealing location length L12 of at least 1%, or with a reduction of increase in the length L11 of the sealing region 270 of at least 1% of the increase of the sum or the length that occurs during the use of conventional translation methods. The sealing region 270 may collapse and the length L11 of the sealing region 270 and the sealing location length L12 may be reduced by these manufacturing approaches.

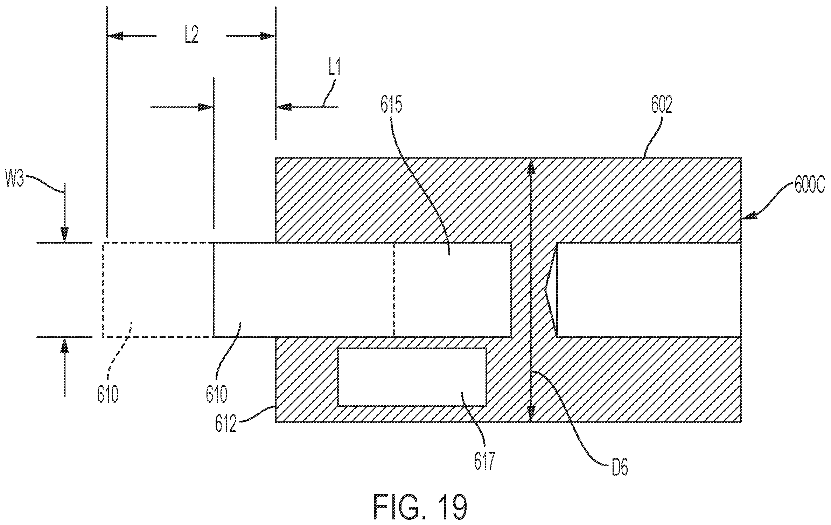

FIG. 19 is an illustration of a retractable tip insertion pin 600C in accordance with some embodiments. As shown, the insertion pin 600C has a pin tip end 610 on the distal end of a retraction member 615. The pin tip end 610 has a diameter W3 that is less than the diameter of the cavity 48 of the stopper 40 (e.g., D12 shown in FIG. 7). For example, the diameter W3 may have a value that is less than or equal to 50% of the diameter of the cavity 48. In further embodiments, the diameter W3 may have a value of between 5% to 50% of the diameter of the cavity 48. In further embodiments, the diameter W3 may have a value of between 15% to 40% of the diameter of the cavity 48 of the stopper 40. Retraction member 615 and the pin tip end 610 are mounted for retraction and reciprocal motion within a body 602. An actuator 617 associated with the insertion pin 600C drives the retraction member 615 and pin tip end 610 between a retracted position (shown in solid lines) and an extended position (shown in broken lines). In the retracted position the pin tip end 610 extends from a shoulder 612 on the body 602 by a length L1. When in the extended position the pin tip end 610 extends from the shoulder 612 of the body 602 by a length L2. In some embodiments, the length L1 of the pin tip end 610 in the retracted position is a length less than the depth L10 of the cavity 48 of the stopper 40. In some embodiments, the length L2 of the pin tip end 610 in the extended position is a length that is greater than or equal to the depth L10 of the cavity 48 of the stopper 40.

Actuator 617 can be any device suitable for driving the retraction member 615 and/or pin tip end 610 between the retracted and extended positions with respect to the body 602. Examples include an electrical device such as a solenoid, or a hydraulic or pneumatic device. Although shown diagrammatically on the body 602 in the illustrated embodiments, the actuator 617 and/or components of the actuator (not shown) associated with the insertion pin 600C can be separate from the body 602. Conventional or otherwise known control systems (not shown) can be used to control the actuator 617.

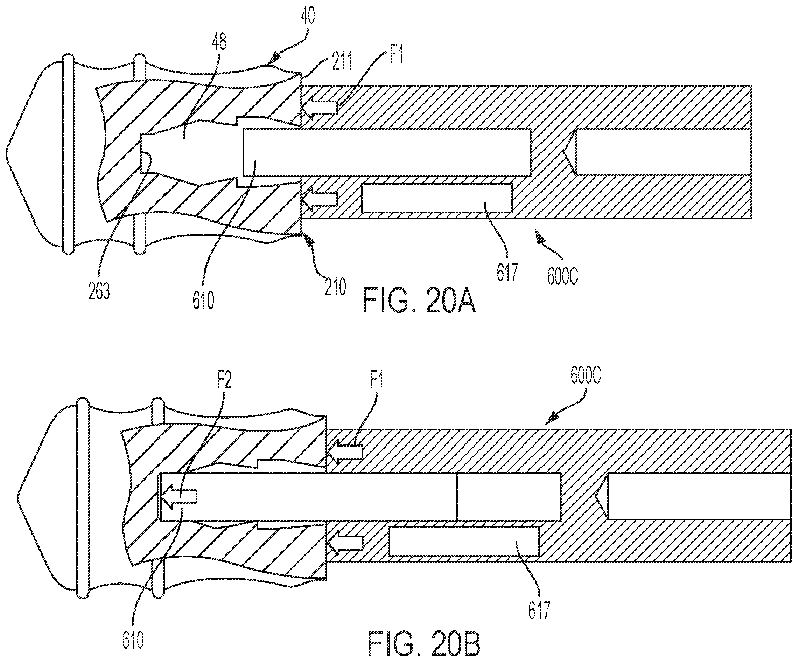

FIG. 20A illustrates the engagement of the insertion pin 600C with the proximal end 210 of the stopper 40 during the assembly of a syringe 10 when the pin tip end 610 is in the retracted position. As shown, the pin tip end 610 is located within the cavity 48 without engaging the distal end portion 261, and the surface of the shoulder 612 engages the annular surface 211 of the stopper 40. Translation forces F1 are thereby applied to the proximal end 210 of the stopper 40. No force components (such as those identified as F2 in FIG. 20B) are applied to the stopper 40 by the insertion pin 600C when the pin tip end 610 is in the retracted position. During the insertion of the pin tip end 610 into the cavity 48 of the stopper 40, and/or during the translation of the insertion pin 600C during the assembly the syringe 10 while the pin tip end 610 is in the retracted position, the pin tip end 610 may enhance the alignment of the stopper 40 and insertion pin 600C.

FIG. 20B illustrates the engagement of the insertion pin 600C with the stopper 40 during the assembly of a syringe 10 when the pin tip end 610 is in the extended position. The pin tip end 610 is thereby located within the cavity 48 and engages the distal end portion 261 of the cavity (e.g., the distal end surface 263) when the surface of the shoulder 612 of the insertion pin 600C engages the annular surface 211 of the stopper 40. Translation forces F2 are thereby applied to the distal end portion 261 of the stopper 40 by the pin tip end 610 while the insertion pin 600C translates the stopper 40. The amount of forces F2 applied to the distal end portion 261 of the cavity 48 by the pin tip end 610 in the extended position may be determined at least in part by the difference between the length L2 of the pin tip end 610 in the extended position and the depth L10 of the cavity. The amount of forces F2 applied by the pin tip end 610 to the stopper 40 during translation of the stopper can thereby be controlled by the actuation of the pin tip end 610 by the actuator 617.

In some embodiments, for example, the forces F2 provided by the pin tip end 610 during translation of the stopper 40 may range from zero to greater than or equal to F1. In some embodiments, insertion force component F2 is a force less than F1. Timing of the application of the insertion force component F2 can also be controlled. For example, in some embodiments, force components F2 may be applied initially (e.g., before the stopper "breaks loose" and begins to slide within the insertion tube 1000), and the force components F2 withdrawn (e.g., reduced to zero by retracting the pin tip end 610) or reduced to a non-zero level. Yet other embodiments may include a pressure sensor capable of monitoring the force components F1 and F2 (not shown), and the actuator 617 can be actuated to retract the pin tip end 610 and withdraw the force component F2 when a pre-determined trigger force is reached. The relative proportions of the force components F1 and F2 can thereby be controlled. The relative timing of the application of the force component F2 with respect to the force F1 during the translation of the stopper 40 can also be controlled. Dynamic relationships between the forces F1 and F2 can be provided during different stages of the translation of the stopper 40 within the insertion tube 1000. For example, the forces F1 and/or F2 applied to the stopper 40 as the stopper 40 is initially inserted into the placement region 1042 the insertion tube 1000 (shown in FIG. 9) may be different than the forces F1 and/or F2 applied to the stopper 40 as the stopper 40 is translated through the transition zone 1040. In some embodiments, other forces F1 and/or F2 may be applied to the stopper 40 as the stopper 40 is translated through the body 1010 of the insertion tube 1000.

Although described in connection with an insertion pin 600C used to translate the stopper 40 through an insertion tube such as 1000, embodiments include a transfer bar insertion pin having a retractable pin such as that of insertion pin 600C, and the use of such a transfer bar insertion pin during the assembly of a syringe 10. By the use of the structures and methods described in connection with FIGS. 19, 20A, and 20B, the stoppers 40 can be translated through tubes during the assembly of the syringe 10 with a reduction of increase in the size, such as the width W2 (FIG. 4), of the microgrooves 133 of the stopper 40. In some embodiments, the reduction of increase in the size, for example in the width W2, of each microgroove 133 may be at least 10% of the increase of the width that occurs during the use of conventional translation methods. In some embodiments the reduction of increase may be at least 15% of the increase of the width that occurs during the use of conventional translation methods. Additionally the stoppers 40 can be translated with a reduction of increase in the sum of the length L11 of the sealing region 270 and the sealing location length L12, or with a reduction of increase in the length L11 of the sealing region 270, of at least 1% of the increase of the sum or the length L11 that occurs during the use of conventional translation methods. In some embodiments, the reduction of increase is at least 3%, and in further embodiments the reduction of increase is at least 5% of the increase of the sum or the length L11 that occurs during the use of conventional translation methods. The sum of the length L11 of the sealing region 270 and the sealing location length L12, or the length L11 of the sealing region 270, may be reduced by these approaches when compared to not using the force concentrators. The sealing region 270 may collapse and the length of the sealing region 270 may be reduced by the assembly and use of the syringe 10 (and cartridge tube 35 of an auto-injector) as described herein.

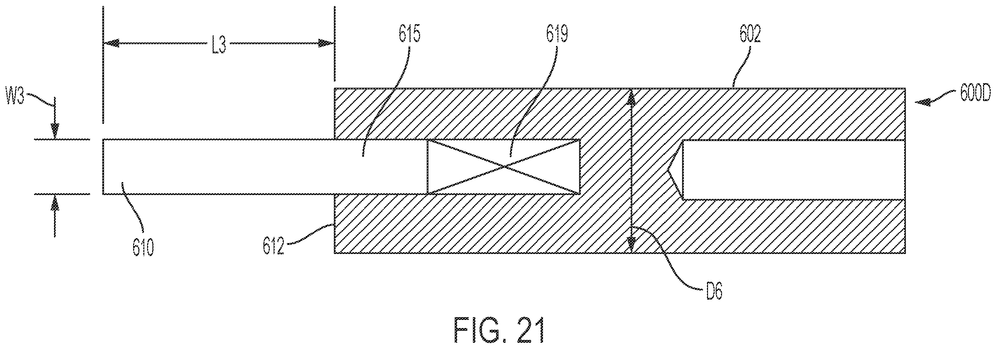

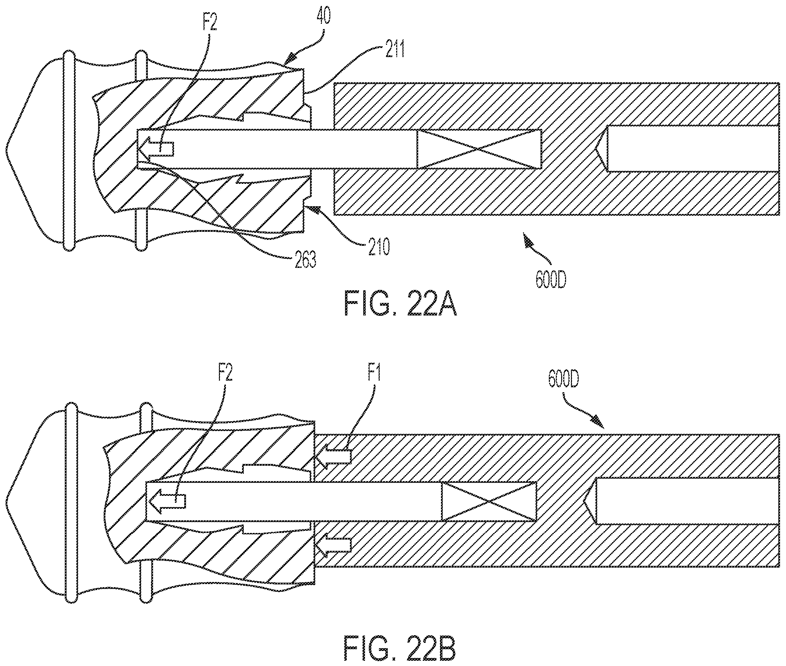

FIG. 21 is an illustration of an insertion pin 600D in accordance with some embodiments. As shown, the insertion pin 600D has a pin tip end 610 on the end of a retraction member 615. The pin tip end 610 has a diameter W3 that is less than the diameter of the cavity 48 of the stopper 40 (e.g., D12 shown in FIG. 7). Retraction member 615 and the pin tip end 610 are mounted for retraction and reciprocal motion within a cavity, for example the cavity 48, in a body 602. A biasing device such as spring 619 biases the retraction member 615 and pin tip end 610 to an extended position shown in FIG. 22A-22B.

FIG. 22A illustrates the engagement of the insertion pin 600D with the proximal end 210 of the stopper 40 during the assembly of a syringe 10 when the pin tip end 610 is in the extended position. FIG. 22B illustrates the engagement of the insertion pin 600D with the stopper 40 during the assembly of a syringe 10 when the pin tip end 610 is in a retracted position. The retraction member 615 and pin tip end 610 can be urged or forced to the retracted position within the body 602 against the bias force exerted by the spring 619. In the extended position, the pin tip end 610 extends from the shoulder 612 of the body 602 by a length L3 which is greater than the depth L10 of the cavity 48 of the stopper 40. As shown in FIG. 22B, in the retracted position the pin tip end 610 extends from the shoulder 612 of the body 602 by a length that is less than or equal to the depth L10 of the cavity 48 of the stopper 40. In some embodiments, the pin tip end 610 is configured to be retractable into the body 602 to such a distance that the length of the pin tip end 610 extending from the body 602 is less than the depth L10 of the cavity 48 to accommodate compliance variations during the manufacturing processes.

As shown in FIG. 22A, as the insertion pin 600D is moved or translated toward engagement with the proximal end 210 of the stopper 40, the pin tip end 610 enters the cavity 48 and engages the distal end portion 261 of the cavity 48 (e.g., the distal end surface 263) before the shoulder 612 of the body 602 engages the annular surface 211 on the proximal end 210 of the stopper 40. By this action the pin tip end 610 may improve the alignment of the stopper 40 and insertion pin 600D. As shown in FIG. 22B, by further advancement or translation of the insertion pin 600D toward the stopper 40, the pin tip end 610 is urged and retracts into the body 602 toward the retracted position against the bias force of the spring 619 as the shoulder 612 of the insertion pin 600D engages the annular surface 211 on the proximal end 210 of the stopper 40. The pin tip end 610 thereby applies a force F2 to the distal end portion 261 of the cavity 48. The amount of the force F2 is determined by the spring 619. By still further advancement of the insertion pin 600D, the insertion pin 600D applies a force F1 to the proximal end 210 of the stopper 40. When the sum of the forces acting on the stopper 40, including F1 and F2, are sufficient to overcome the frictional forces acting on the stopper 40, the stopper 40 will translate through the insertion tube 1000. In some embodiments, force F2 is less than force F1. In some embodiments, the insertion pin 600D may operate differently than insertion pin 600C described above, for example, by providing a constant force F2 on the stopper 40 throughout the period of translation of the stopper 40 while the force F1 is applied.

Although described in connection with an insertion pin 600D used to translate the stopper 40 through an insertion tube such as 1000, embodiments include a transfer bar insertion pin having a biased pin such as that of insertion pin 600D, and the use of such a transfer bar insertion pin during the assembly of a syringe 10. By the use of the structures and methods described in connection with FIGS. 21, 22A, and 22B, the stoppers 40 can be translated through tubes during the assembly of the syringe 10 with a reduction in increase of the size, for example the width W2 (FIG. 4), of the microgrooves 133 of the stopper 40. In some embodiments, the reduction of increase in the size, for example in the width W2, of each microgroove 133 may be at least 10% of the increase of the width that occurs during the use of conventional translation methods. In some embodiments the reduction of increase may be at least 15%. Additionally, by the use of the structures and methods described in connection with FIGS. 21, 22A, and 22B, the stoppers 40 can be translated through tubes during the assembly of the syringe 10 with a reduction of increase in the sum of the length L11 of the sealing region 270 and the sealing location length L12, or with a reduction of increase in the length L11 of the sealing region 270, of at least 1% of the increase of the sum or the length L11 that occurs during the use of conventional translation methods. In further embodiments, the stoppers 40 can be translated through tubes during assembly of the syringe 10 with a reduction of increase in the sum of the length L11 of the sealing region 270 and the sealing location L12, or the length L11 of the sealing region 270, of at least 3%. In further embodiments, the reduction of increase may be at least 5% of the increase of the sum or the length L11 that occurs during the use of conventional translation methods. The sum of the length L11 of the sealing region 270 and the sealing location length L12, and/or the length L11 of the sealing region 270, may be reduced by these approaches. The sealing region 270 may collapse and the length of the sealing region 270 may be reduced by these manufacturing approaches, in comparison to when these manufacturing approaches are not used.

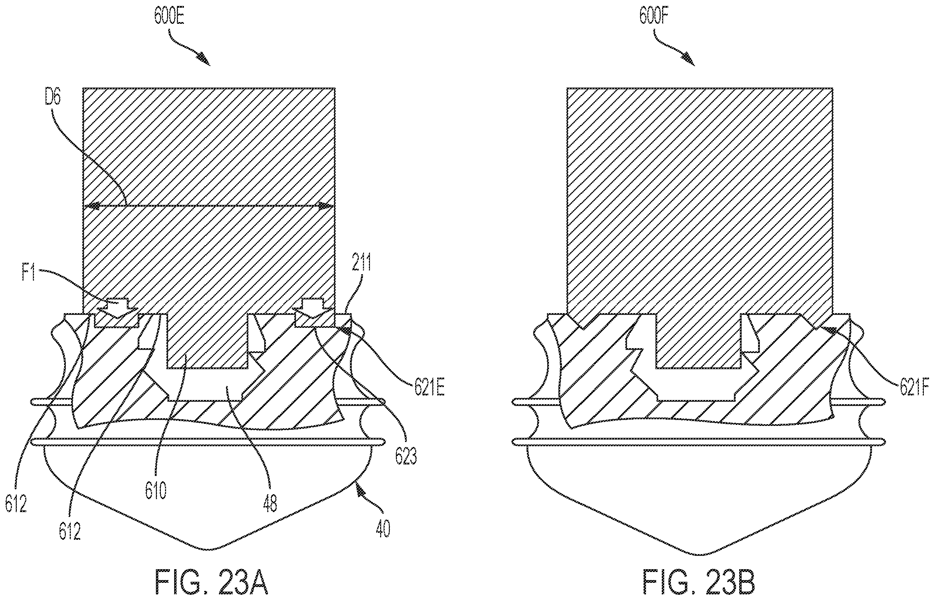

FIGS. 23A-23D illustrate insertion pins 600E-600H, respectively, in accordance with some embodiments. As shown, insertion pins 600E-600H include examples of force concentrators 621E-621H, respectively, extending from the shoulder 612 around the pin tip end 610. Force concentrators 621E-621H are located to engage the annular surface 211 on the proximal end 210 of the stopper 40 during the assembly of syringes 10. As the insertion pins 600E-600H are moved toward the stoppers 40 during the assembly of the syringes 10, surfaces 623, respectively, of the force concentrators 621E-621H will engage portions of the annular surfaces 211 on the stoppers 40 before other surface portions of the shoulder 612 engage the proximal ends of the stoppers 40. Force concentrators 621E-621H have widths less than the width of the area of the insertion pins 600E-600H peripheral to the pin tip end 610, and less than a width of the annular surface 211 (e.g., the portion of distal end portion 261 peripheral to the cavity 48). Force concentrators 621E-621H thereby concentrate the application of forces F1 at the locations on the annular surface 211 engaged by the force concentrators. The force concentrators are configured for biasing the forces that are exerted into the outer diameter of the stopper 40 which may reduce, or eliminate, the amount that which the microgrooves 133 open upon compression.

In some embodiments, only the force concentrators 621E-621H engage the proximal ends 210 of the stoppers 40 during the translation of the stoppers 40, and in such embodiments the force F1 is applied to the annular surface 211 of the stoppers only at the locations engaged by the force concentrators 621E-621H. In other embodiments, insertion pins 600E-600H may be configured to cause portions of the shoulder 612 in addition to the force concentrators 621E-621H to engage the proximal ends 210 of stoppers 40 during the translation of the stoppers 40, and in such embodiments, forces in addition to the concentrated forces F1 applied by the force concentrators 621E-621H are applied to the stoppers by the insertion pins 600E-600H to cause translation of the stoppers 40.

Force concentrators 621E-621H are generally annular or ring-shaped when viewed from a distal end of the insertion pins 600E-600H. In some embodiments, the force concentrators 621E-621H extend continuously around the pin tip end 610. In other embodiments, the force concentrators 621E-621H are discontinuous, and include a plurality of spaced-apart sections (not shown in FIGS. 23A-23D) extending around the pin tip end 610 that engage the annular surface 211 of the stopper 40 at a plurality of discrete locations around the cavity 48. The height of the force concentrators 621E-621H (e.g., with respect to surfaces of the portions of the shoulder 612 from which they extend) and the locations of the force concentrators 621E-621H (e.g., with respect to the location on the annular surfaces 211 of the stoppers engaged by the force concentrators) and/or profile or shape of the force concentrators 621E-621H can be configured to provide the desired amounts of concentrated forces F1 at desired locations on the stoppers 40.

FIG. 23A illustrates a force concentrator 621E having a generally flat surface 623 in accordance with some embodiments. In the embodiments illustrated in FIG. 23A, the force concentrator 621E is generally rectangular in cross section. The width of the surface 623 of the force concentrator 621E can be configured to provide the desired concentrated forces F1. In the illustrated embodiments, the concentrated forces F1 are provided at locations on the annular surface 211 inwardly from the outer peripheral edge of the stopper 40. In other embodiments the force concentrator 621E shown in FIG. 23A may be located to apply concentrated forces at the periphery of the annular surface 211 of the stopper 40.

FIG. 23B illustrates a force concentrator 621F having a generally knife-edge or sharp surface 623 in accordance with some embodiments. In the embodiments illustrated in FIG. 23B the force concentrator 621F is generally triangular in cross section. The slopes of the surface 623 of the force concentrator 621F can be configured to provide the desired concentrated forces F1. The peak of the force concentrator 621F is located on the insertion pin 600F to engage the annular surface 211 of the stopper 40 at a location between the outer edge of the cavity 48 and the outer edge of the annular surface 211.

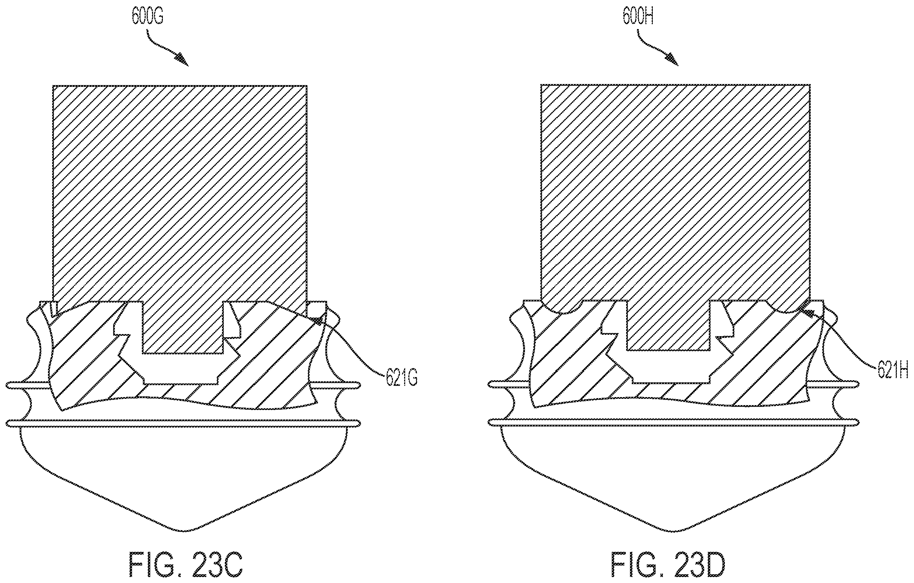

FIG. 23C illustrates a force concentrator 621G having a generally knife-edge or sharp surface 623 in accordance with some embodiments. Force concentrator 621G is similar to the force concentrator 621F described above, but the peak of the force concentrator 621G is located on the insertion pin 600G to engage the annular surface 211 of the stopper 40 at the outer edge portion of the annular surface 211.

FIG. 23D illustrates a force concentrator 621H having a generally radiused or convex surface 623.

Although described in connection with insertion pins 600E-600H used to translate the stopper 40 through an insertion tube such as 1000, embodiments include a transfer bar insertion pin having force concentrators substantially the same as or similar to those of force concentrators 621E-621H, and the use of such a transfer bar insertion pin during the assembly of a syringe 10. Similarly, force concentrators substantially the same as or similar to those of force concentrators 621E-621H can be incorporated onto the shoulder 312 of the plunger rod 50 (e.g., as described above in connection with FIGS. 8A and 8B and as shown in FIGS. 25A-25D) in addition to or as an alternative to the force concentrators 330. The force concentrators 621E-621H can also be incorporated into the insertion pins 600A-600D described above in connection with FIGS. 17-19, 20A, 20B, 21, 22A, and 22B. By the use of the structures and methods described in connection with FIGS. 23A-23D, the stoppers 40 can be translated through tubes during the assembly of the syringe 10 with a reduction of increase of the size, for example the width W2 (FIG. 4), of the microgrooves 133 of the stopper 40. For example, the reduction of increase of the width W2 of the microgrooves 133 may be at least 10% of the increase of the width that occurs from the use of conventional translation methods. In further examples, the reduction of increase of the width W2 may be at least 15% of the increase of the width that occurs from the use of conventional translation methods. Additionally, in use the structures and methods described may cause the stoppers 40 to be translated through tubes during the assembly of the syringe 10 with a reduction of increase in the sum of the length L11 of the sealing region 270 and the sealing location length L12, or with a reduced increase in the length L11 of the sealing region 270, of at least 1% of the increase of the sum or the length L11 that occurs during the use of conventional translation methods. In further embodiments, the reduction of increase in the sum of the length L11 of the sealing region 270 and the sealing location length L12, or just the length L11 of the sealing region 270, of at least 3%, or in further embodiments, at least 5% of the increase of the sum or the length L11 that occurs during the use of conventional translation methods. The sum of the length L11 of the sealing region 270 and the sealing location length L12, and/or the length L11 of the sealing region 270, may be reduced by these approaches. The sealing region 270 may collapse and the length of the sealing region 270 may be reduced by these manufacturing approaches. As previously described, the size of the microgrooves 133 may be reduced by these approaches, as well.

FIGS. 24A-24D illustrate stoppers 40A-40D, respectively, in accordance with some embodiments. As shown, stoppers 40A-40D include force concentrators 47A-47D, respectively, extending from portions of the annular surfaces 211 on the proximal ends 210 around the cavities 48. While shown in Figs. 24A-24D as being used separately, the force concentrators 47A-47D may be used in combination on a single stopper. For example, in one example, force concentrators 47A and 47B may both be used on a single stopper 40. In another example, any combination of force concentrators 47A-47D may be used on the stopper 40. Force concentrators 47A-47D are located to engage the distal surfaces of structures used to translate the stoppers 40 through tubes during the assembly and use of syringes 10 including the stoppers 40. For example, force concentrators 47A-47D can be engaged by the distal ends such as shoulders 612 of insertion pins 600 and/or shoulders of the transfer bar insertion pins during the assembly of syringes 10, and/or by the shoulders 312 of plunger rods 50 during the use of the syringes 10 (e.g., as described in connection with FIGS. 10A and 10B and illustrated in FIGS. 25A-25D). As the structures that engage and translate the stoppers 40A-40D are moved toward the stoppers during the assembly of the syringes 10, surfaces on the distal ends of the structures will engage the force concentrators 47A-47D before engaging other portions of the annular surface 211 on the distal ends of the stoppers 40A-40D. Force concentrators 47A-47D have widths less than the widths of the portions of annular surface 211 peripheral to the cavity 48. Force concentrators 47A-47D thereby concentrate the application of forces F1 at the locations of the force concentrators 47A-47D when engaged by the structures translating the stoppers 40A-40D, respectively, during the assembly and/or use of the syringes 10. In some embodiments, only the force concentrators 47A-47D are engaged by the structures translating the stoppers 40A-40D, respectively, during the assembly and/or use of the syringes 10, and the forces F1 are applied to proximal ends 210 of the stoppers only at the locations of the force concentrators 47A-47D. In other embodiments, structures translating the stoppers 40A-40D engage portions of the annular surfaces 211 on the proximal ends 210 in addition to the force concentrators 47A-47D during the translation of the stoppers 40, and in such embodiments, forces in addition to the concentrated forces F1 applied through the force concentrators 47A-47D are applied to the stoppers 40 by the structures to cause translation of the stoppers 40.

Force concentrators 47A-47D are generally annular or ring-shaped when viewed from a proximal ends of the stoppers 40A-40D. In some embodiments, the force concentrators in FIGS. 24A-24D extend continuously around the proximal ends 210 of the stoppers 40A-40D. In other embodiments, the force concentrators in FIGS. 24A-24D are discontinuous and include a plurality of spaced-apart sections (not shown in FIGS. 24A-24D) extending around the proximal ends 210 that are engaged by the translating structures at a plurality of discrete locations. In other embodiments, the force concentrators 47A-47D may be present on the distal end portion 308 of the plunger rod 50 as depicted in FIGS. 25A-25D.

It is to be appreciated that the force concentrators depicted in FIGS. 25A-25D are the same or substantially the same shape and size as the force concentrators shown in FIGS. 23A-23D and/or FIGS. 24A-24D. The heights of the force concentrators 47A-47D, 621E-621H (e.g., with respect to annular surfaces 211 from which they extend), locations of the force concentrators (e.g., with respect to the cavity 48 and/or outer edges of the annular surfaces 211) and/or profile or shape of the force concentrators may be configured to provide a desired amount of concentrated forces F1 at desired locations on the stoppers.

FIG. 24A illustrates a force concentrator 47A having a surface 49 that is generally flat in accordance with some embodiments. In the embodiments illustrated in FIG. 24A, the force concentrator 47A is generally rectangular in cross section. The width of the surface 49 of the force concentrator 47A can be configured to provide the desired concentrated forces F1. In some embodiments, the concentrated forces F1 are provided at locations on the annular surface 211 inwardly from the outer peripheral edge of the annular surface 211. In other embodiments the force concentrator 47A may be located to apply concentrated forces at the periphery of the annular surface 211 of the stopper 40A.

FIG. 24B illustrates a force concentrator 47B having the surface 49, wherein the surface 49 is generally knife-edge or a sharp surface in accordance with some embodiments. In the embodiments illustrated in FIG. 24B the force concentrator 47B is generally triangular in cross section. The slopes of the surface 49 of the force concentrator 47B can be configured to provide the desired concentrated forces F1. The peak of the force concentrator 47B is located between the outer edge of the cavity 48 and the outer edge of the annular surface 211.

FIG. 24C illustrates a force concentrator 47C having the surface 49 that is generally knife-edge or sharp in accordance with some embodiments. Force concentrator 47C is similar to the force concentrator 47B described above, but the peak of the force concentrator 47C is located at the outer edge portion of the annular surface 211.

FIG. 24D illustrates a force concentrator 47D wherein the surface 49 is generally radiused or convex.

By the use of the structures and methods described in connection with FIGS. 24A-24D, the stoppers 40A-40D can be translated through tubes during the assembly of the syringe 10 with a reduction of increase in the size, for example the width W2 (FIG. 4), of the microgrooves 133 of the stopper 40. For example, the reduction of increase of the width W2 of the microgrooves 133 may be at least 10% of the increase of the width that occurs during the use of conventional translation methods. In further examples, the reduction of increase of the width W2 may be at least 15% of the increase of the width that occurs during the use of conventional translation methods. Additionally, by the use of the structures and methods described in connection with FIGS. 24A-24D, the stoppers 40A-40D can be translated through tubes during the assembly of the syringe 10 with a reduction of increase in the sum of the length L11 of the sealing region 270 and the sealing location length L12, or with a reduced increase in the length L11 of the sealing region 270, of at least 1% of the increase of the sum or the length L11 that occurs during the use of conventional translation methods. In further embodiments, the reduction of increase may be at least 3%, and in further embodiments, the reduction of increase may be at least 5%. The sum of the length L11 of the sealing region 270 and the sealing location length L12, or the length L11 of the sealing region 270, may be reduced by these approaches. The sealing region 270 may collapse and the length of the sealing region 270 may be reduced by these manufacturing approaches.

With reference to FIGS. 25A-25D, additional embodiments of plunger rods with force concentrators for use in the translating the stopper 40 (FIG. 7) are shown. Specifically, FIG. 25A illustrates the plunger rod 50 with the tip 310 extending from the distal end portion 308 of the plunger rod 50. Further, the plunger rod 50 includes force concentrators features 330A extending from the distal end portion 308 and positioned adjacent the tip 310. As illustrated, the force concentrators 330A have a square or rectangular shape, however, various other configurations may be incorporated. For example, FIG. 25B illustrates an additional embodiment of the plunger rod 50 having force concentrators 330B, illustrated as the annular edge portion of the distal portion 308 positioned around the pin tip 310.

FIG. 25C illustrates an additional variation of the plunger rod 50 having force concentrators 330C. In the illustrative embodiment of FIG. 25C, the force concentrators 330C are illustrated as triangular protrusions extending from an end of the distal portion 308 of the plunger rod 50.

FIG. 25D illustrates an additionally embodiment the plunger rod 50 having force concentrators 330D. In this embodiment, the force concentrators 330D are illustrated as rounded protrusions extending from the distal portion 308 of the plunger rod 50. However, the shape and configuration of the force concentrators 330A-330D may be any variety of shapes including, but not limited to, a flat surface, a linear taper, curvilinear, rounded, or multiple tapers.

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims.

**Claims**

1. A method comprising:

placing a distal end (215) of a stopper (40, 40A-40D, 65) on a proximal end (1012) of an insertion tube (1000), the insertion tube and the stopper being silicone free, the stopper including a plunger rod engaging cavity (48) and a sealing region (270) having a length (L11) spaced from a proximal end (210) of the stopper by a sealing location length (L12), the sealing region having at least one rib (42, 44) including at least one microgroove (133) in a polymer barrier (140), the at least one microgroove having an initial width (W2);

positioning an insertion pin (600, 600B-600H) on a proximal end of the stopper without contacting a distal region of the plunger rod engaging cavity, wherein the insertion pin has a cylindrical body (602) that includes a distal end (606) having a shoulder (612) and a pin tip end (610) that has a diameter smaller than a diameter of the plunger rod engaging cavity;

contacting the proximal end of the stopper with the shoulder of the insertion pin, wherein the shoulder of the insertion pin comprises at least one force concentrating feature (621E-621H) including an annular structure (623) extending from the shoulder (612) of the insertion pin, optionally including one of at least a flat, sharp or radiused surface configured to engage the proximal end of the stopper; and

applying a force to the at least one concentrating feature such that the force (F1)_applied to the stopper is applied to an annular surface (211) of the proximal end of the stopper such that the force is biased towards an outer diameter of the stopper.

2. The method of claim 1, wherein the method further includes guiding the stopper (40, 40A-40D, 65) through an entire length of the insertion tube (1000) and into a syringe barrel (20), the syringe barrel being silicone free.

3. The method of claim 2, wherein during the guiding of the stopper (40, 40A-40D, 65) through the insertion tube (1000), the insertion pin (600C, 600D) engages the distal end region (261) of the plunger rod engaging cavity (48).

4. The method of any one of the preceding claims, wherein during the step of placing the distal end (215) of the stopper (40, 40A-40D, 65) on the proximal end (1012) of the insertion tube (1000), the stopper is in an uncompressed state.

5. The method of any one of claims 2-4, wherein during the step of guiding the stopper (40, 40A-40D, 65) through the insertion tube (1000), the stopper is in a compressed state.

6. The method of any one of the preceding claims, wherein applying the force on the proximal end (608) of the insertion pin (600, 600B-600H) further includes transferring at least a portion of the force onto the proximal end (210) of the stopper (40, 40A-40D, 65).

7. The method of claim 6, wherein transferring at least a portion of the force onto the proximal end (210) of the stopper (40, 40A-40D, 65) includes applying one or both of (1) a first force to the proximal end of the stopper, or (2) a second force to the distal region (261) of the plunger rod engaging cavity (48).


**Patentansprüche**

1. Verfahren, umfassend:

Platzieren eines distalen Endes (215) eines Stopfens (40, 40A-40D, 65) an einem proximalen Ende (1012) eines Einführrohres (1000), wobei das Einführrohr und der Stopfen silikonfrei sind, wobei der Stopfen einen Kolbenstangeneingriffshohlraum (48) und einen Dichtungsbereich (270) mit einer Länge (L11) beabstandet von einem proximalen Ende (210) des Stopfens um eine Dichtungsstellenlänge (L12) beinhaltet, wobei der Dichtungsbereich zumindest eine Rippe (42, 44) aufweist, die zumindest eine Mikronut (133) in einer Polymerbarriere (140) beinhaltet, wobei die zumindest eine Mikronut eine Anfangsbreite (W2) aufweist;

Positionieren eines Einführstiftes (600, 600B-600H) an einem proximalen Ende des Stopfens, ohne einen distalen Bereich des Kolbenstangeneingriffshohlraums zu kontaktieren, wobei der Einführstift einen zylindrischen Körper (602) aufweist, der ein distales Ende (606) mit einer Schulter (612) und ein Stiftspitzenende (610) beinhaltet, das einen Durchmesser aufweist, der kleiner als ein Durchmesser des Kolbenstangeneingriffshohlraums ist;

Kontaktieren des proximalen Endes des Stopfens mit der Schulter des Einführstiftes, wobei die Schulter des Einführstiftes zumindest ein Kraftkonzentrationsmerkmal (621E-621H) umfasst, das eine ringförmige Struktur (623) beinhaltet, die sich von der Schulter (612) des Einführstiftes erstreckt, optional beinhaltend eines von zumindest einer flachen, scharfen oder abgerundeten Oberfläche, die dazu konfiguriert ist, das proximale Ende des Stopfens in Eingriff zu nehmen; und

Aufbringen einer Kraft auf das zumindest eine Konzentrationsmerkmal, sodass die Kraft (F1), die auf den Stopfen aufgebracht wird, auf eine ringförmige Oberfläche (211) des proximalen Endes des Stopfens aufgebracht wird, sodass die Kraft zu einem Außendurchmesser des Stopfens vorgespannt ist.

2. Verfahren nach Anspruch 1, wobei das Verfahren ferner Führen des Stopfens (40, 40A-40D, 65) durch eine gesamte Länge des Einführrohres (1000) und in einen Spritzenzylinder (20) beinhaltet, wobei der Spritzenzylinder silikonfrei ist.

**3.** Verfahren nach Anspruch 2, wobei während des Führens des Stopfens (40, 40A-40D, 65) durch das Einführrohr (1000) der Einführstift (600C, 600D) den distalen Endbereich (261) des Kolbenstangeneingriffshohlraums (48) in Eingriff nimmt.

**4.** Verfahren nach einem der vorhergehenden Ansprüche, wobei während des Schrittes des Platzierens des distalen Endes (215) des Stopfens (40, 40A-40D, 65) an dem proximalen Ende (1012) des Einführrohres (1000) der Stopfen in einem unkomprimierten Zustand ist.

**5.** Verfahren nach einem der Ansprüche 2-4, wobei während des Schrittes des Führens des Stopfens (40, 40A-40D, 65) durch das Einführrohr (1000) der Stopfen in einem komprimierten Zustand ist.

**6.** Verfahren nach einem der vorhergehenden Ansprüche, wobei das Aufbringen der Kraft auf das proximale Ende (608) des Einführstiftes (600, 600B-600H) ferner Übertragen von zumindest einem Teil der Kraft auf das proximale Ende (210) des Stopfens (40, 40A-40D, 65) beinhaltet.

**7.** Verfahren nach Anspruch 6, wobei das Übertragen von zumindest einem Teil der Kraft auf das proximale Ende (210) des Stopfens (40, 40A-40D, 65) Aufbringen von einem oder beidem von (1) einer ersten Kraft auf das proximale Ende des Stopfens oder (2) einer zweiten Kraft auf den distalen Bereich (261) des Kolbenstangeneingriffshohlraums (48) beinhaltet.

**Revendications**

**1.** Procédé comprenant :

le placement d'une extrémité distale (215) d'un bouchon (40, 40A à 40D, 65) sur une extrémité proximale (1012) d'un tube d'insertion (1000), le tube d'insertion et le bouchon étant exempts de silicone, le bouchon comprenant une cavité de mise en prise de tige de piston (48) et une zone d'étanchéité (270) présentant une longueur (L11) espacée d'une extrémité proximale (210) du bouchon par une longueur d'emplacement d'étanchéité (L12), la zone d'étanchéité comportant au moins une nervure (42, 44) comprenant au moins une micro-rainure (133) dans une barrière polymère (140), ladite au moins une micro-rainure présentant une largeur initiale (W2) ;
le positionnement d'une broche d'insertion (600, 600B à 600H) sur une extrémité proximale du bouchon sans entrer en contact avec une zone distale de la cavité de mise en prise de tige de piston, ladite broche d'insertion possédant un corps cylindrique (602) qui comprend une extrémité distale (606) comportant un épaulement (612) et une extrémité de pointe de broche (610) qui présente un diamètre inférieur au diamètre de la cavité de mise en prise de tige de piston ;
la mise en contact de l'extrémité proximale du bouchon avec l'épaulement de la broche d'insertion, ledit épaulement de la broche d'insertion comprenant au moins un élément de concentration de force (621E à 621H) comprenant une structure annulaire (623) s'étendant à partir de l'épaulement (612) de la broche d'insertion, comprenant éventuellement une parmi au moins une surface plate, pointue ou arrondie conçue pour mettre en prise l'extrémité proximale du bouchon ; et
l'application d'une force sur ledit au moins un élément de concentration de sorte que la force (F1) appliquée sur le bouchon soit appliquée sur une surface annulaire (211) de l'extrémité proximale du bouchon de sorte que la force soit sollicitée vers un diamètre externe du bouchon.

**2.** Procédé selon la revendication 1, ledit procédé comprenant en outre le guidage du bouchon (40, 40A à 40D, 65) sur toute une longueur du tube d'insertion (1000) et à l'intérieur d'un corps de seringue (20), le corps de seringue étant exempt de silicone.

**3.** Procédé selon la revendication 2, pendant le guidage du bouchon (40, 40A à 40D, 65) à travers le tube d'insertion (1000), ladite broche d'insertion (600C, 600D) venant en prise avec la zone d'extrémité distale (261) de la cavité de mise en prise de tige de piston (48).

**4.** Procédé selon l'une quelconque des revendications précédentes, pendant l'étape de placement de l'extrémité distale (215) du bouchon (40, 40A à 40D, 65) sur l'extrémité proximale (1012) du tube d'insertion (1000), ledit bouchon se trouvant dans un état non comprimé.

**5.** Procédé selon l'une quelconque des revendications 2 à 4, pendant l'étape de guidage du bouchon (40, 40A à 40D, 65) à travers le tube d'insertion (1000), ledit bouchon se trouvant à l'état comprimé.

**6.** Procédé selon l'une quelconque des revendications précédentes, ladite application de la force sur l'extrémité proximale (608) de la broche d'insertion (600, 600B à 600H) comprenant en outre le transfert d'au moins une partie de la force sur l'extrémité proximale (210) du bouchon (40, 40A à 40D, 65).

**7.** Procédé selon la revendication 6, ledit transfert d'au moins une partie de la force sur l'extrémité proximale (210) du bouchon (40, 40A à 40D, 65) comprenant l'application d'une ou des deux forces parmi (1) une première force sur l'extrémité proximale du bouchon, ou (2) une seconde force sur la zone distale (261) de la cavité de mise en prise de tige de piston (48).

**20**

FIG. 1A

FIG. 1B

FIG. 2

FIG. 3

FIG. 4          FIG. 5          FIG. 6

FIG. 7

FIG. 8A

FIG. 8B

50

302

306

308

312

314

310

318

D16

L9

308

312

310

D13

FIG. 9

600

612

610

602

608

606

## FIG. 10A

L1

W3

602

600

612

D6

606

608

## FIG. 10B

FIG. 11A

FIG. 11B

FIG. 11C

FIG. 11D

FIG. 12A          FIG. 12B          FIG. 12C          FIG. 12D

EP 4 247 459 B1

FIG. 13A

FIG. 13B

EP 4 247 459 B1

EP 4 247 459 B1

FIG. 13C

FIG. 13D

FIG. 14

FIG. 15

FIG. 16

FIG. 17

FIG. 18

FIG. 19

FIG. 20A

FIG. 20B

FIG. 21

EP 4 247 459 B1

FIG. 22A

FIG. 22B

EP 4 247 459 B1

FIG. 23A

FIG. 23B

FIG. 23C

FIG. 23D

FIG. 24A     FIG. 24B     FIG. 24C     FIG. 24D

FIG. 25A

FIG. 25B

FIG. 25C

FIG. 25D

**EP 4 247 459 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020112612 A
- WO 2020118275 A
- EP 3058975 A
- EP 3342439 A
- EP 3409311 A
- WO 2017123835 A
- US 5708044 A, Branca
- US 6541589 B, Baillie
- US 7531611 B, Sabol
- US 8637144 B, Ford
- US 9139669 B, Xu